# EUROPEAN PATENT APPLICATION

(11) **EP 4 696 345 A2**
(43) Date of publication of application: **18.02.2026**
(21) Application number: 25206953.9
(22) Date of filing: 30.11.2021
(51) Int. Cl.: A61M 5/315

(54) **ADMINISTRATION DEVICE FOR IN PARTICULAR INTRAVITREAL ADMINISTRATION OF A FLUID**

(30) Priority: 01.12.2020 EP 20211106
(62) Divisional of application: 21815541.4
(71) Applicant: Boehringer Ingelheim International GmbH, 55216 Ingelheim am Rhein (DE)
(72) Inventor: ROTH, Axel, 55216 Ingelheim am Rhein (DE); COLLINGS, Ralph Donald Quentin, Chepston, NP16 7NN (GB); LABAT-ROCHECOUSTE, Andrew Guy, Bristol, BS31 1AU (GB); SHARP, Nicholas James, Bristol, BS6 5TD (GB); SCHUY, Steffen, 55216 Ingelheim am Rhein (DE)
(74) Representative: Von Rohr Patentanwälte Partnerschaft mbB

(57) **Abstract**

An administration device for intravitreal administration of a fluid is proposed, wherein a second actuation part of the administration device is configured to axially block a plunger rod and/or a first actuation part of the administration device, wherein the second actuation part is to be actuated in order to axially release the plunger rod and/or the first actuation part such that the first actuation part can be actuated. The administration device comprises a securing member, wherein the securing member is configured to block a back-movement of the plunger rod and/or the first actuation part at least in an initial position and/or in an administration position of the plunger rod and wherein the securing member is configured to block actuation of the second actuation part before reaching the administration position of the plunger rod.

## Description

The present invention relates to an administration device for preferably ophthalmic, in particular intravitreal, administration of a fluid according to the preamble of claim 1.

The present invention relates generally to the field of administration/delivery devices for pharmaceutical administration/delivery of a fluid, in particular a drug, to a patient, i.e. a human or animal.

The administration/delivery device according to the present invention is preferably adapted to deliver, in particular inject, a drug (directly) into the eye, in particular the vitreous, interior and/or posterior chambers of the eye, of a human or animal. Thus, the present invention in particular relates to administration/delivery devices for ophthalmic, in particular intravitreal, injection of a fluid, in particular a drug. However, the administration device according to the present invention can also be used for administration/injection of a fluid, in particular a drug, into other parts of the human or animal body.

WO 2014/005728 A1 relates to a small volume syringe suitable for ophthalmic injections, wherein the syringe is pre-filled with a drug and comprises a body, a stopper and a plunger. The plunger comprises a plunger contact surface arranged to contact the stopper such that the plunger can be used to push the stopper towards an outlet, thereby delivering a fluid contained within the syringe.

The object of the present invention is to provide an improved - in particular solely mechanical - administration/delivery device which is user-friendly and/or easy, intuitive and/or safe to use and/or minimizes or at least reduces the risk of misapplication, misuse, contamination, incorrect and/or imprecise dosing, and/or of the delivery/administration of air bubbles and/or minimizes or at least reduces the requirements and/or complexity of or within the manufacturing processes of the administration/delivery device.

The above object is achieved by an administration/delivery device according to claim 1. Advantageous further developments are subject of the dependent claims.

The administration/delivery device, hereinafter referred to as administration device, according to the present invention is preferably adapted to (immovably) hold an in particular cylindrical container for a fluid, in particular a drug.

The container is preferably pre-filled with the fluid, in particular a drug.

Preferably, a stopper/piston/plunger, hereinafter referred to as stopper, is movably arranged within the container, in particular such that by an axial movement of the stopper towards the outlet of the container the fluid contained in the container is dispensed.

Preferably, the administration device comprises the container and/or the stopper.

Further, the administration device comprises a retainer/holder, hereinafter referred to as retainer, preferably wherein the container is or can be in particular immovably held/received by and/or at least partially arranged within the retainer.

The administration device comprises an actuation mechanism for actuating the administration device and/or for moving the stopper within the container, in particular for so-called priming of the administration device before administering the fluid and/or for dispensing/administering the fluid out of the container, as will be explained in the following.

The administration device, in particular its actuation mechanism, comprises a plunger/piston/connecting rod - in particular directly and/or axially - acting on the stopper, a first actuation part - in particular directly and/or axially acting on the plunger/piston/connecting rod - and a second actuation part, wherein the second actuation part is movably, in particular rotatably, attached to the retainer and/or wherein the plunger/piston/connecting rod, hereinafter referred to as plunger rod, and/or the first actuation part are/is in particular axially movable, in particular slidable, relative to the retainer and/or container.

Preferably, the plunger rod can be axially moved relative to the retainer and/or container, in particular (further) into the container, by actuating, in particular depressing, the first actuation part, in particular in order to prime the administration device and/or - subsequently to priming - to dispense/administer the fluid out of the container.

The actuation mechanism of the administration device, in particular the first actuation part, is preferably configured to (axially) move, in particular push, the plunger rod and/or the stopper further into the container and/or from an initial/unactuated position to an administration/ready-to-use/primed position during priming, prior to administration and/or by (manually) actuating, in particular depressing, the first actuation part, in particular for the first time, and/or from the administration/ready-to-use/primed position to a final/actuated/end-of-use position after priming, during (subsequent) administration and/or by- in particular manually - actuating the first actuation part, in particular again and/or for the second time.

According to one aspect of the present invention, the second actuation part is configured to axially block - in particular in a form-fitting manner - the plunger rod and/or the first actuation part, in particular from being moved (directly) into the administration/ready-to-use/primed position and/or into the final/actuated/end-of-use position and/or from being (completely) actuated and/or inserted into the container, wherein the second actuation part is to be (manually) actuated, in particular rotated or slided, in order to axially release/unblock the plunger rod and/or the first actuation part, such that the first actuation part can be - in particular partially, completely and/or subsequently - actuated and/or such that the plunger rod and/or the stopper can be moved from the initial/unactuated position to the administration/ready-to-use/primed position and/or from the administration/ready-to-use/primed position to the final/actuated/end-of-use position and/or further into the container.

In this way, an easy, intuitive and user-friendly usage of the administration device is achieved. In particular, the priming process is clearly separated from the administering process, thereby derisking misuse of the administration device, fluid waste and/or incorrect/imprecise dosing.

Preferably, the second actuation part can be actuated by turning/rotating or sliding the second actuation part relative to the container and/or retainer and/or around the plunger rod, mostly preferred by at least essentially 90 degrees. In this way, the usability is improved and/or the risk of misuse is decreased.

Since the second actuation part is preferably actuated in a direction at least essentially transversal to the direction of actuation of the first actuation part, a possible impact of the actuation of the second actuation part on the first actuation part and/or on the injection side is minimized or at least reduced.

The first actuation part can be actuated by (manually) pushing, in particular depressing, the first actuation part towards the second actuation part and/or towards the retainer.

By actuating, in particular pushing/depressing, the first actuation part and, thereby, moving the plunger rod and/or the stopper, it is possible to (manually) prime the administration device and/or to push out air/gas contained within the container and/or a needle attached thereto.

In the context of the present invention, the term "prime" / "priming" preferably refers to the (necessary) preparation of the administration device (immediately) prior to administration of the fluid contained in the administration device, in particular its container.

Preferably, during priming, the plunger rod is (axially) moved such that and/or until the plunger rod, in particular its front portion and/or tip, abuts the stopper.

Mostly preferred, during priming, the plunger rod is (axially) moved such that the plunger rod, in particular its front portion and/or tip, moves/pushes the stopper - in particular towards an outlet - and/or such that a (small) part of the fluid is dispensed and/or air, i.e. air bubbles contained in the fluid, the container and/or a needle attached thereto, is/are removed, in particular pushed out.

The administration device is preferably held vertically during priming, in particular such that the outlet of the container and/or a needle attached thereto points upwards and/or that air can be at least partially pushed out of the container.

Preferably, the volume contained in the container after priming corresponds to a desired/pre-defined dose to be administered to a patient/user.

Typically, the volume of the fluid contained in the (pre-filled) container exceeds the volume of the dose to be administered so that air contained in the fluid, container and/or the needle attached thereto can be removed at least partially by priming without effecting the dose to be administered.

However, priming of the administration device does not imply that a container is actually filled with a fluid and/or that a part of the fluid is dispensed. In particular, the actuation mechanism, mostly preferred the first actuation part, can also be actuated even if the container is empty and/or without moving and/or acting on the stopper.

In other words, "prime" / "priming" in particular means a movement of the plunger rod and/or the stopper from the initial/unactuated position to a defined position, hereinafter referred to as administration/ready-to-use/primed position, in particular before administering the fluid and/or by (partially) actuating the first actuation part, in particular for the first time.

Accordingly, all actions performed prior to administering the fluid and/or actuating the second actuation part are preferably to be considered as priming and/or the priming process.

Priming is preferably done/completed solely by (partially) actuating, in particular pushing, the first actuation part, mostly preferred manually.

In the context of the present invention, the term "administer" / "administration" preferably relates to the (actual) delivery/dispensing of the fluid from the administration device, preferably its container, mostly preferred through an injection needle attached thereto.

While it is preferred that the administration device according to the present invention is used for administering a fluid, in particular a drug, directly to the body of a patient, e.g. by using an injection needle and/or by injecting a fluid directly into the eye of a patient, in particular the vitreous body, the term "administer" and "administration" also refers to delivery/dispensing the fluid without a patient being present.

Preferably, "administer" / "administration" means and/or includes a movement of the plunger rod and/or the stopper from the administration/ready-to-use/primed position to a final/actuated/end-of-use position, in particular after priming and/or after actuating the first actuation part for the first time, mostly preferred by actuating the first actuation part completely and/or for the second time.

The initial/unactuated position, hereinafter referred to as the initial position, is preferably the (axial) position of the plunger rod and/or the stopper relative to the retainer and/or container before priming of the administration device and/or actuating the first actuation part (for the first time) and/or in the initial state of the administration device.

In the initial state of the administration device and/or when the plunger rod and/or the stopper are/is in the initial position, the first actuation part and the second actuation part are preferably unactuated.

It is preferably only possible to (directly) actuate either the first actuation part or the second actuation part in the initial state of the administration device and/or when the plunger rod and/or the stopper are/is in the initial position.

Mostly preferred, it is only possible to (directly) actuate the first actuation part in the initial state of the administration device and/or when the plunger rod and/or the stopper are/is in the initial position.

Preferably, the second actuation part is blocked in the initial position/state. It is thus preferably not possible to (directly) actuate the second actuation part in the initial state of the administration device and/or when the plunger rod and/or the stopper are/is in the initial position.

The plunger rod and/or the stopper can be moved out of the initial position and/or from the initial position to the administration/ready-to-use/primed position in particular solely by (partially) actuating the first actuation part for the first time.

The administration/ready-to-use/primed position, hereinafter referred to as administration position, is preferably the (axial) position of the plunger rod and/or the stopper relative to the retainer and/or the container (immediately) after priming and/or (immediately) after actuating, in particular pushing/depressing, the first actuation part for the first time and/or before administration is carried out and/or in the primed state of the administration device.

The primed state of the administration device is preferably the state of the administration device immediately after actuating the first actuation part for the first time and/or immediately after reaching the administration position. In the primed state of the administration device, the first actuation part is only partially actuated and/or the second actuation part is (still) unactuated.

In the administration position, the plunger rod and/or the stopper are/is arranged closer to an outlet of the administration device, in particular the container, compared to the initial position.

In particular, the plunger rod and/or the stopper are/is inserted further into the container in the administration position compared to the initial position.

The plunger rod and/or the first actuation part are/is preferably blocked from being completely actuated and/or moved from the initial position directly/continuously to the final position, mostly preferred by means of the second actuation part. With other words, the plunger rod and/or the first actuation part can preferably only be actuated and/or moved directly/continuously from the initial position to the administration position.

Preferably, the second actuation part is configured to axially block/stop the plunger rod and/or the first actuation part in the administration position and/or from being moved directly/continuously from the initial position to the final position.

In the primed state of the administration device and/or immediately after reaching the administration position and/or after (partially) actuating the first actuation part, the administration device, in particular the plunger rod and/or the first actuation part, are/is blocked from further movement/actuation, in particular by means of the second actuation part.

Preferably, the second actuation part is to be actuated after priming and/or after reaching the administration position and/or in the primed state of the administration device in order to unblock the administration device and/or to axially release the plunger rod and/or the first actuation part, in particular such that the first actuation part can be - in particular again and/or completely and/or for the second time - actuated and/or such that the plunger rod can be moved further to the final position and/or such that administration can be carried out.

With other words, the second actuation part is preferably to be actuated in order to switch the state of the administration device, in particular the first actuation part, from a blocked state into the administration/ready-to-use/unblocked state.

In the primed/blocked state and/or immediately after reaching the administration position, the second actuation part is preferably in a blocking position in which the second actuation part axially blocks the plunger rod and/or the first actuation part, mostly preferred such that the first actuation part cannot be further and/or completely actuated.

By actuating, in particular turning/rotating or sliding, the second actuation part, the second actuation part is moved from the blocking position to a releasing position in which the second actuation part axially releases the plunger rod and/or the first actuation part and/or in which the first actuation part can be further and/or completely actuated.

In the administration/ready-to-use/unblocked state of the administration device and/or after actuating the second actuation part, the administration device is ready to use, the first actuation part can be further/completely actuated, the plunger rod can be moved to the final position and/or the fluid can be administered to a patient, in particular without performing further steps apart from actuating the first actuation part.

It is preferably not possible to reverse the actuation of the first actuation part in the administration position/state and/or in the final position/state.

It is preferably not possible to (again) actuate the second actuation part in the initial state/position, during movement of the plunger rod from the initial position to the administration position and/or before reaching the administration position and/or to reverse the actuation of the second actuation part after actuating the second actuation part and/or in the releasing position of the second actuation part.

The final/actuated/end-of-use position, hereinafter referred to as final position, is preferably the (axial) position of the plunger rod and/or the stopper relative to the retainer (immediately) after administration and/or after (completely) actuating the first actuation part, in particular for the second time, and/or in the final/actuated/end-of-use state of the administration device, hereinafter referred to as final state.

In the final state of the administration device and/or when the plunger rod and/or the stopper are/is in the final position, the administration has been carried out and/or the first actuation part is (completely) actuated.

In the final state/position, the plunger rod and/or the stopper are/is arranged closer to the outlet of the administration device compared to the initial state/position and/or the administration position/state.

In particular, no further fluid can be dispensed/administered in the final state and/or after the final position is reached.

It is preferably not possible to (again) actuate and/or reverse the actuation of the first actuation part and/or the second actuation part in the final state and/or after the final position is reached.

Due to the construction of the administration device, in particular the (mechanical) interaction of the parts/components of the administration device, a user/practitioner is guided through the usage of the administration device, thereby reducing the risk of misapplication, misuse, contamination, incorrect and/or imprecise dosing.

According to another aspect of the present invention which can be realized also independently, the administration device, in particular the actuation mechanism, comprises a securing member/part, wherein the securing member/part is configured to (axially) secure the plunger rod and/or the first actuation part and/or to (axially) block a back-movement of the plunger rod and/or the first actuation part, in particular a movement of the plunger rod and/or the first actuation part out of the container and/or in a direction opposite to the direction of actuation, at least in the initial position and/or in the administration position of the plunger rod. Further, the securing member/part is configured to block the second actuation part, in particular actuating/rotating the second actuation part, in the initial position of the plunger rod and/or before, in particular (only) until, reaching the administration position of the plunger rod and/or during priming and/or movement of the plunger rod from the initial position to the administration position.

In this way, the risk of misuse/misapplication of the administration device is reduced.

The securing member/part, hereinafter referred to as securing member, is preferably embodied as an in particular flexible ring that is arranged within a receptacle of the retainer.

Preferably, the securing member is coupled loosely and/or with radial play to the plunger rod in the initial position and/or in the administration position and/or is radially pretensioned against and/or tightly coupled to the plunger rod during movement of the plunger rod from the initial position towards the administration position.

The securing member is preferably configured to (radially) engage the plunger rod and/or to (axially) block the plunger rod and/or the first actuation part, in particular a back-movement of the plunger rod and/or the first actuation part, at least in the initial position and/or in the administration position of the plunger rod.

Additionally, the securing member is preferably configured to (radially) engage the second actuation part and/or to block actuation/rotation of the second actuation part, in particular in the initial position and/or during movement from the initial position to the administration position, mostly preferred only until the plunger rod reaches the administration position.

Preferably, the securing member automatically disengages/unblocks the second actuation part upon actuating the first actuation part, during movement of the plunger rod from the initial position to the administration position, in particular when the administration position is reached and/or after completing priming.

The plunger rod preferably comprises or forms a ramp for the securing member, preferably wherein the ramp is configured to tension/widen the securing member upon actuation of the first actuation part and/or during movement of the plunger rod from the initial position towards the administration position.

The plunger rod preferably comprises an indentation for the securing member.

Mostly preferred, the plunger rod, in particular its indentation, is configured to at least partially, preferably completely, release the securing member and/or to disengage the securing member from the second actuation part upon actuating the first actuation part, during movement of the plunger rod from the initial position to the administration position, in particular when the administration position is reached and/or after completing priming, mostly preferred such that the second actuation part is unblocked and/or can be actuated.

The actuation mechanism of the administration device is preferably configured to move the securing member from an engaging position to a disengaging position, in particular automatically.

The engaging position is preferably the (radial) position of the securing member relative to the second actuation part in the initial position of the plunger rod, during priming of the administration device and/or actuating the first actuation part and/or until reaching the administration position.

In the engaging position, the securing member engages the second actuation part radially and/or blocks rotational actuation of the second actuation part and/or in a direction of actuation.

The disengaging position is preferably the (radial) position of the securing member relative to the second actuation part after priming of the administration device and/or actuating the first actuation part (for the first time) and/or (immediately) after reaching the administration position.

In the disengaging position, the securing member is disengaged from the second actuation part radially and/or no longer blocks rotational actuation of the second actuation part and/or in a direction of actuation.

Thus, the second actuation part cannot be actuated when the securing member is in the engaging position and can only be actuated when the securing member is in the disengaging position.

The securing member is preferably moved from the engaging position to the disengaging position and/or snaps into the indentation of the plunger rod automatically, by spring force and/or suddenly/abruptly and/or audibly.

In this way, an audible/haptic feedback, in particular an audible click, is provided that clearly indicates the end of priming and/or when the administration position is reached.

In the context of the present invention, the term "audible" preferably refers to a sound that can be heard by an average human ear with normal hearing. An audible sound/feedback preferably comprises a sound level above the absolute threshold of hearing. For example, a sound with frequencies between 0,2 kHz and 5 kHz and a sound pressure between 20 dB and 100 dB is to be considered as audible.

The above-mentioned aspects and features of the present invention and the aspects and features of the present invention that will become apparent from the claims and the following description can in principle be implemented independently from one another, but also in any combination or order.

Further aspects, advantages, features and properties of the present invention will become apparent from the claims and the following description of a preferred embodiment with reference to the drawings, in which:
- Fig. 1: is a schematic perspective view of a proposed administration device;
- Fig. 2: is an exploded view of the administration device according to Fig. 1;
- Fig. 3: is an exploded view of the administration device, rotated by 90 degrees compared to Fig. 2;
- Fig. 4A: is a schematic longitudinal section of the administration device in the initial position/state;
- Fig. 4B: is a schematic longitudinal section of the administration device in the administration position/state before actuating a second actuation part of the administration device;
- Fig. 4C: is a schematic longitudinal section of the administration device in the administration position/state after actuating the second actuation part;
- Fig. 4D: is a schematic longitudinal section of the administration device in the final position/state;
- Fig. 5A: is a schematic longitudinal section of the administration device in the initial position/state, rotated by 90 degrees about its main axis compared to Fig. 4A;
- Fig. 5B: is a schematic longitudinal section of the administration device in the administration position/state before actuating the second actuation part, rotated by 90 degrees about its main axis compared to Fig. 4B;
- Fig. 5C: is a schematic longitudinal section of the administration device in the administration position/state after actuating the second actuation part, rotated by 90 degrees about its main axis compared to Fig. 4C;
- Fig. 5D: is a schematic longitudinal section of the administration device in the final position/state, rotated by 90 degrees about its main axis compared to Fig. 4D;
- Fig. 6A: is a perspective partial section of the administration device in the initial position/state;
- Fig. 6B: is a perspective partial section of the administration device in the administration position/state before actuating the second actuation part;
- Fig. 7A: is a schematic cross-section of the administration device in the administration position/state before actuating the second actuation part;
- Fig. 7B: is a schematic cross-section of the administration device in the administration position/state after actuating the second actuation part;
- Fig. 8A: is a perspective partial section of the administration device in the administration position/state before actuating the second actuation part; and
- Fig. 8B: is a perspective partial section of the administration device in the administration position/state after actuating the second actuation part.

In the figures, the same reference signs are used for the same or similar parts and components, resulting in corresponding or comparable properties, features and advantages, even if these properties, features and advantages are not repeatedly described.

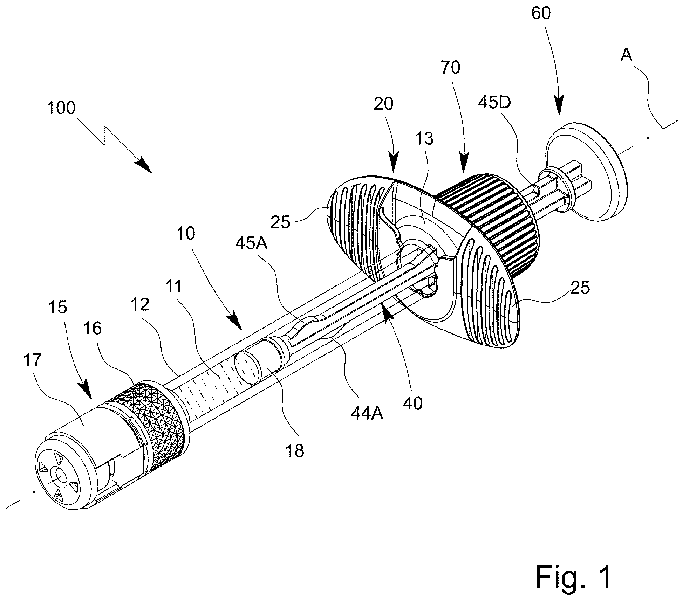

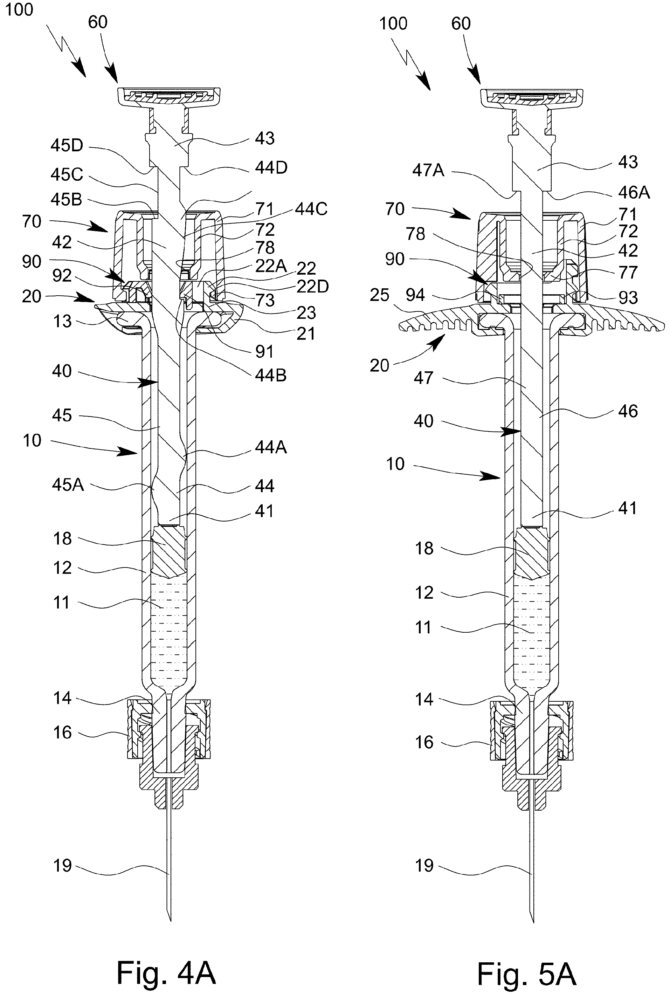

In the following, the construction and the components/parts of a proposed administration device 100 will be explained with reference to Figs. 1 to 3. The interaction of the components/parts and the operation of the administration device 100 will be explained with reference to Figs. 4A-4D, 5A-5D, 6A-6B, 7A-7B and 8A-8B, wherein - for reasons of simplicity - some reference signs have been omitted although the related features/components/parts are illustrated.

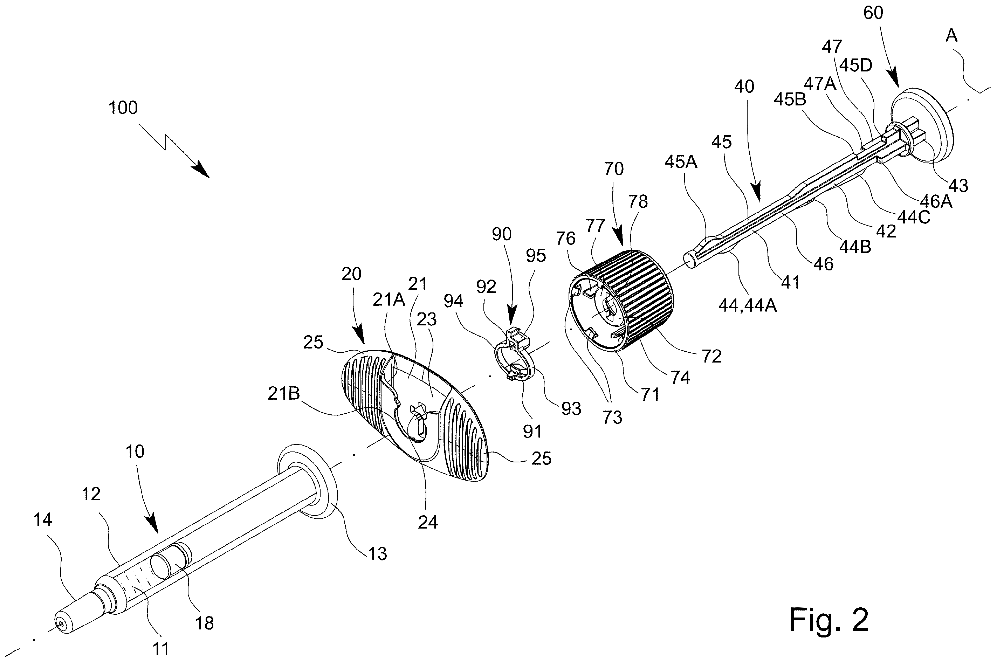

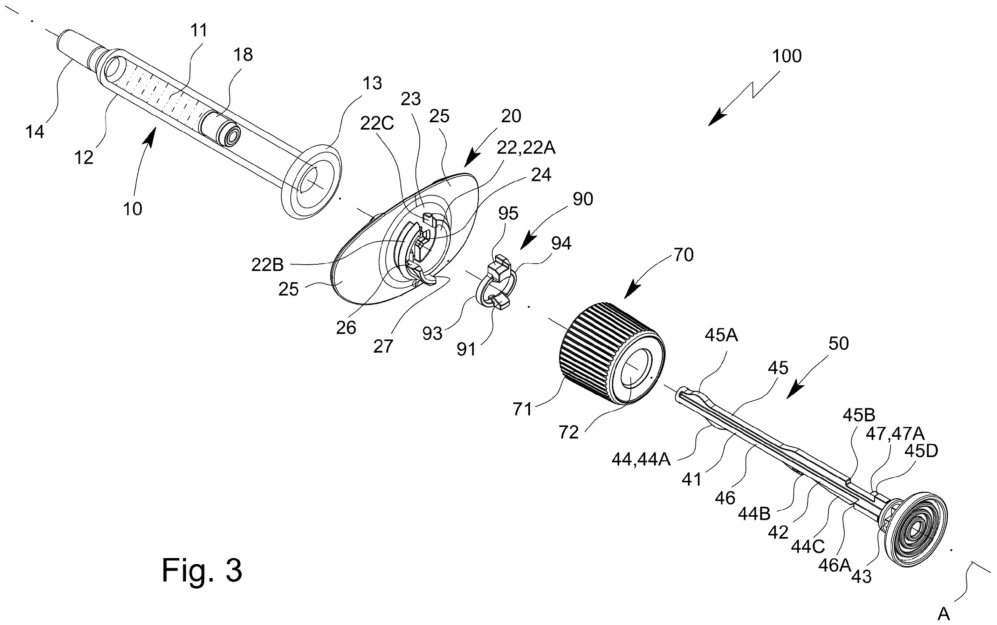

Fig. 1 is a schematic perspective view of the proposed administration device 100 in the initial position/state. Fig. 2 and Fig. 3 are exploded views of the administration device 100 from different angles.

The administration device 100 is of multi-part construction and/or comprises multiple parts/components, preferably wherein some or all parts of the administration device 100 are connected to each other by means of a snap/clip-on connection and/or in a screwless manner.

The terms "snap", "clip" and/or "snap/clip-on connection" is preferably understood to refer to an assembly/connection method, wherein at least two parts/components are assembled/connected to one another - in particular in a force-fitting and/or form-fitting manner - using the elasticity/flexibility of the parts/components to be assembled/connected and/or without using (additional) fastening elements, such as nails, screws, bolts or the like.

As best seen in Figs. 2 and 3, the administration device 100 preferably comprises a container 10, a retainer 20 and/or an actuation mechanism having a plunger rod 40, a first actuation part 60, a second actuation part 70 and/or a securing member 90.

Preferably, some or all parts/components of the administration device 100, in particular the container 10, the retainer 20, the plunger rod 40, the first actuation part 60, the second actuation part 70 and/or the securing member 90 are made out of, in particular injection molded from, plastics, mostly preferred polyethylene and/or polypropylene.

The administration device 100 is preferably elongated and/or shaped as a syringe.

In particular, the administration device 100 is embodied as a handheld device and/or shaped in such a manner that it ergonomically fits into a user's / practitioner's hand.

The administration device 100, in particular the container 10, the plunger rod 40, the first actuation part 60 and/or the second actuation part 70, is/are preferably at least essentially cylindrical and/or rotationally symmetric.

Preferably, the administration device 100, in particular the container 10, the plunger rod 40, the first actuation part 60 and/or the second actuation part 70, comprises/comprise and/or defines/define a main axis A, in particular wherein the main axis A is a central, longitudinal, symmetry and/or rotation axis of the administration device 100, in particular the container 10, the plunger rod 40, the first actuation part 60 and/or the second actuation part 70, and/or wherein the main axis A centrally runs through the administration device 100, in particular the container 10, the retainer 20, the plunger rod 40, the first actuation part 60, the second actuation part 70 and/or the securing member 90.

In the following, spatial orientations, positions and/or arrangements, in particular the terms "radial" or "radially" and/or "axial" or "axially", refer to the main axis A unless otherwise stated.

Preferably, the first actuation part 60 forms a (first) axial end and/or a rear end and the container 10, in particular its tip/outlet, forms a second (axial) end and/or a front end of the administration device 100.

In the normal position of use, the administration device 100, in particular the container 10, preferably points away from a user/practitioner and/or towards a patient and the first actuation part 60 points towards a user/practitioner and/or away from a patient.

As already noted in the introductory part, the administration device 100 is adapted to administer/dispense a fluid 11, in particular a drug, to a patient, i.e. a human or animal.

Preferably, the container 10 is pre-filled with the fluid 11 and/or already contains the fluid 11 in the initial state of the administration device 100.

The administration device 100 is preferably adapted to administer/dispense the fluid 11 out of the container 10, in particular its outlet, to a patient, mostly preferred by means of its actuation mechanism, as will be explained later in greater detail.

The container 10 is preferably elongated and/or embodied as a hollow cylinder.

Preferably, the container 10 comprises a main body 12, preferably wherein the main body 12 is embodied as a hollow cylinder and/or wherein the inner diameter of the main body 12 is constant over the longitudinal extension of the main body 12.

The container 10, in particular its main body 12, is preferably formed integrally / in one piece. Mostly preferred, the container 10, in particular its main body 12, is made out of glass or injection molded from plastics.

The container 10 preferably comprises a first axial end 13 and a second axial end 14.

The first axial end 13 of the container 10 is preferably embodied as a flange/collar and/or comprises an enlarged outer diameter compared to the outer diameter of the main body 12 and/or the second axial end 14.

The second axial end 14 of the container 10 is preferably embodied as the tip of the container 10 and/or comprises or forms the outlet of the container 10.

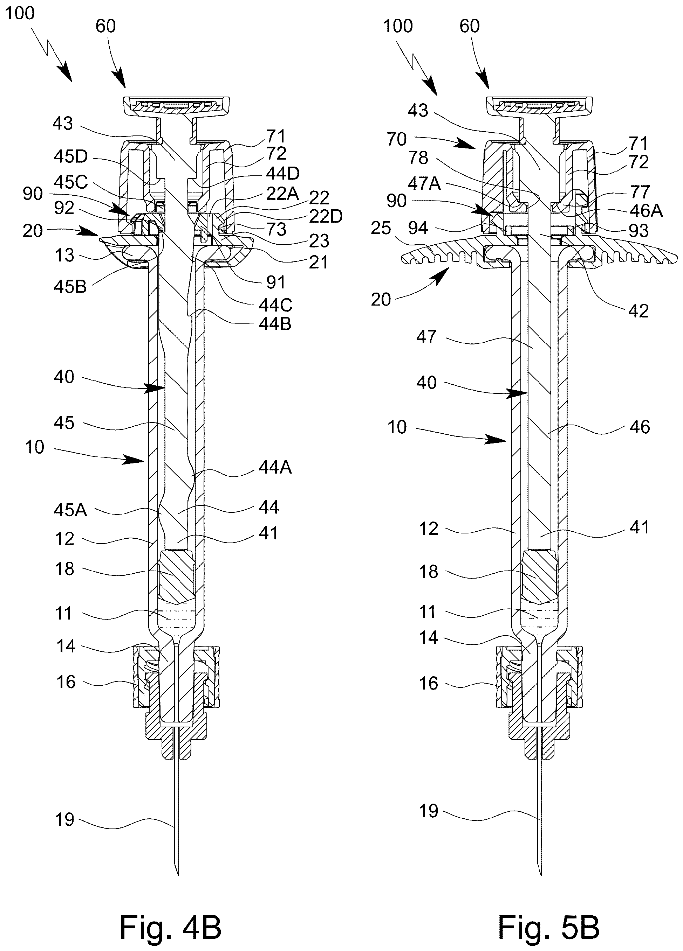

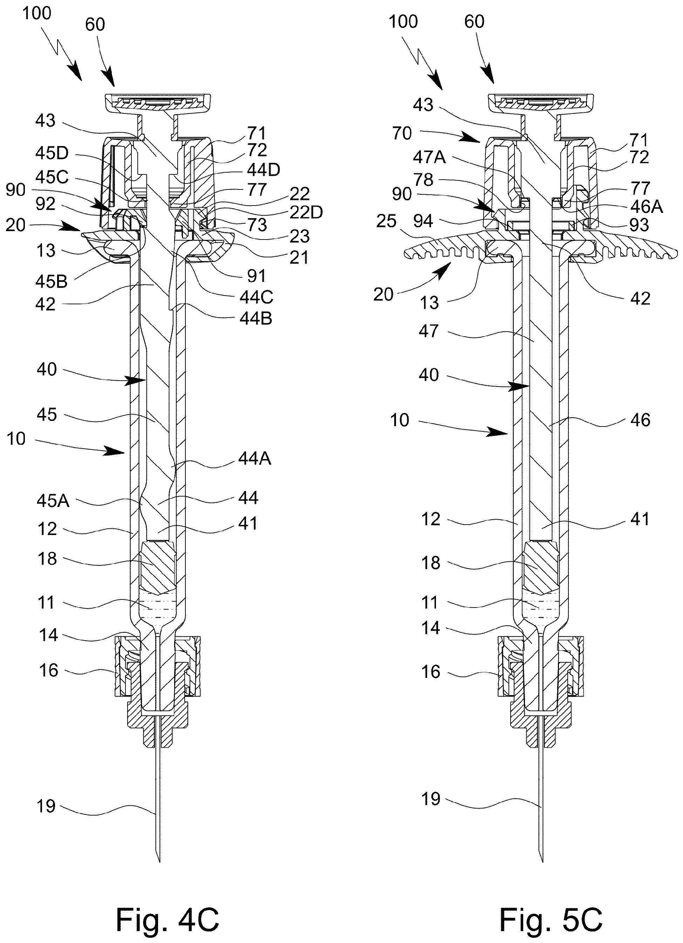

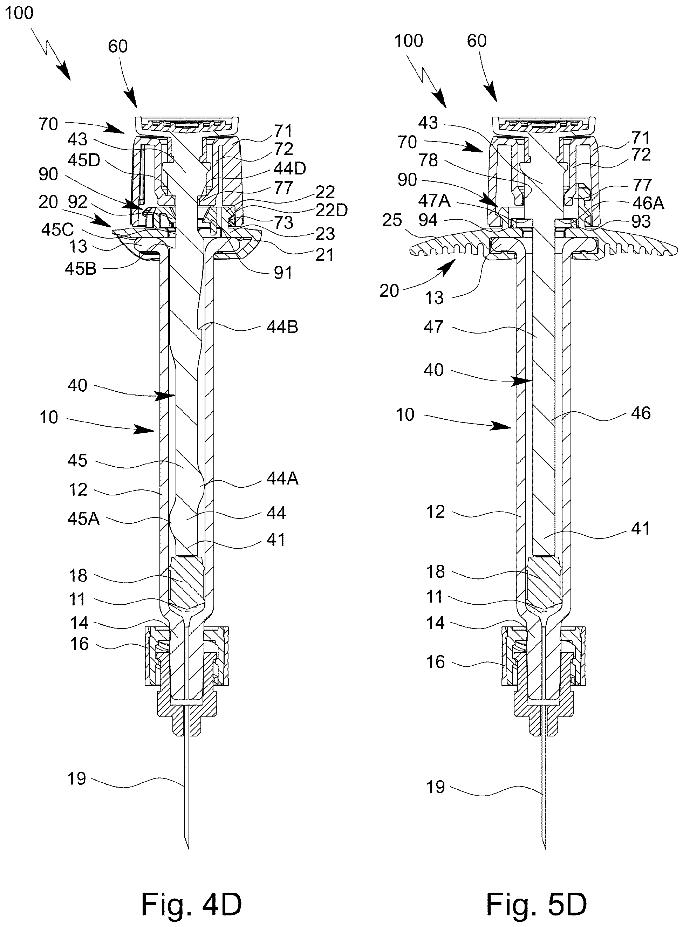

As best seen in Figs. 3 and 4, the second axial end 14 of the container 10 is preferably cone-shaped and/or tapered. Mostly preferred, the second axial end 14 comprises a smaller inner and/or outer diameter compared to the inner and/or outer diameter of the main body 12 and/or the first axial end 13.

Preferably, the second axial end 14 of the container 10 is embodied as a connection part for a needle 19 (not shown in Figs. 1 to 3).

As shown in Fig. 1, the administration device 100, in particular the container 10, is optionally equipped with a closure/lock 15, in particular a Luer lock, in order to protect/seal the administration device 100, in particular the container 10, mostly preferred its second axial end 14, and/or to provide a connection for the needle 19.

Preferably, the needle 19 can be attached to the container 10, in particular the second axial end 14, by means of the lock 15, mostly preferred in a form-fitting and/or force-fitting manner.

The lock 15 preferably comprises a connector 16 and/or a cap 17, preferably wherein the connector 16 and/or the cap 17 are attached to the second axial end 14 of the container 10, in particular in a form-fitting and/or force-fitting manner, and/or wherein the cap 17 axially covers the second axial end 14 and/or the connector 16, mostly preferred in the initial state of the administration device 100.

The connector 16 is preferably adapted to connect the needle 19 to the container 10, in particular its second axial end 14.

In order to connect the needle 19 to the container 10, the cap 17 can be removed from the container 10 and/or the connector 16. Subsequently, the needle 19 can be attached to, in particular screwed into, the connector 16.

Preferably, the lock 15, in particular the connector 16, comprises an internal thread such that the needle 19 can be screwed into the lock 15, in particular the connector 16. In this way, a particularly firm connection of the needle 19 and lock 15 can be achieved. However, other constructional solutions are possible as well.

The administration device 100, in particular the container 10, preferably comprises a stopper 18, preferably wherein the stopper 18 is movably arranged within the container 10, in particular its main body 12.

The main body 12 of the container 10 and the stopper 18 preferably delimit a chamber for the fluid 11.

The stopper 18 is preferably made out of, in particular injection molded from, plastic, in particular an elastomer.

The stopper 18 is preferably cylindrical and/or comprises an outer diameter that corresponds at least essentially to and/or is (slightly) larger than the inner diameter of the container 10, in particular the main body 12, preferably such that the stopper 18 presses against the inner wall of the container 10, in particular the main body 12.

Preferably, the stopper 18 sealingly abuts the inner wall of the container 10, in particular the main body 12, and/or comprises or forms at least one (radial) seal, in particular such that the fluid 11 cannot leak out and/or flow through a gap between the stopper 18 and the inner wall of the main body 12.

By moving/pushing the stopper 18 towards the second axial end 14 of the container 10, preferably by means of the actuation mechanism, in particular by means of the first actuation part 60, the volume of the chamber delimited by the main body 12 and by the stopper 18 can be reduced, thereby delivering the fluid 11 contained in the container 10.

As already mentioned, the administration device 100 preferably comprises the retainer 20, preferably wherein some or all parts/components of the administration device 100, are (directly) mounted on and/or - in particular either rigidly/immovably or movably - attached to or held by the retainer 20.

Thus, the retainer 20 preferably functions as a supporting/connecting/mounting structure for the other parts/components of the administration device 100, in particular the container 10, the plunger rod 40, the first actuation part 60, the second actuation part 70 and/or the securing member 90.

Preferably, the container 10 is rigidly/immovably held by and/or attached to the retainer 20.

Preferably, the plunger rod 40, the first actuation part 60, the second actuation part 70 and/or the securing member 90 are/is movably attached to the retainer 20, as will be explained later in further detail.

As best seen in Figs. 2 and 3, the retainer 20 preferably comprises a - preferably radially/laterally and/or axially open - mount/receptacle/socket 21 for the container 10 and/or a - preferably radially/laterally and/or axially open - support/holder/frame 22 for the first actuation part 60, the second actuation part 70 and/or the securing member 90.

The mount/receptacle/socket 21, hereinafter referred to as mount 21, and the support/holder/frame 22, hereinafter referred to as support 22, are preferably made out of the same material and/or formed, in particular injection molded, in one piece and/or from plastic.

The retainer 20, in particular the mount 21, is preferably adapted to immovably hold the container 10, preferably in a clamped manner and/or in particular in such a way that the container 10 is radially and axially held within and/or by means of the retainer 20, in particular the mount 21.

The container 10, in particular its first axial end 13, is preferably radially/laterally inserted, in particular pressed/clipped, into the retainer 20, mostly preferred the mount 21.

To this end, the inner shape/contour of the mount 21 preferably corresponds at least essentially to the outer shape/contour of the container 10, in particular its first axial end 13.

The mount 21 is preferably slot-shaped and/or comprises a radial opening 21A and/or an axial opening 21B, preferably wherein the container 10, in particular its axial end 13, is radially (inserted), in particular pressed/clipped, into the mount 21 via radial opening 21A and/or wherein the main body 12 of the container 10 extends out of the mount 21 via axial opening 21B.

The retainer 20, in particular the support 22, is preferably adapted to (axially) receive and/or (radially) support/guide the plunger rod 40, the first actuation part 60, the second actuation part 70 and/or the securing member 90, in particular such that the second actuation part 70 can be rotated relative to the retainer 20 and/or is axially held by the retainer 20, in particular the support 22, and/or such that the plunger rod 40 and/or the first actuation part 60 can axially move relative to and/or through the retainer 20, in particular the support 22, as will be explained in the following.

The plunger rod 40, the first actuation part 60, the second actuation part 70 and/or the securing member 90 are/is preferably axially attached to and/or inserted into the retainer 20, mostly preferred the support 22.

The retainer 20, in particular the support 22, preferably comprises or forms an in particular at least essentially cylindrical socket/receptacle 22A for the securing member 90 and/or the second actuation part 70, preferably wherein the support 22, in particular the socket/receptacle 22A is axially open.

The retainer 20, in particular the support 22, preferably comprises an in particular ring-shaped wall 22B, preferably wherein the wall 22B defines and/or radially delimits the socket/receptacle 22A, hereinafter referred to as receptacle 22A.

Preferably the support 22, in particular the wall 22B is radially open and/or comprises a radial opening 22C.

The securing member 90 is preferably inserted into the receptacle 22A and/or is radially surrounded by the wall 22B.

It is preferred that the securing member 90 radially protrudes out of the receptacle 22A and/or through the radial opening 22C, as will be explained later in further detail.

The mount 21 and the support 22 are preferably arranged on opposite sides of the retainer 20.

The retainer 20 preferably comprises an intermediate/separating wall 23, hereinafter referred to as intermediate wall 23, preferably wherein the intermediate wall 23 is arranged between the mount 21 and the support 22 and/or (axially) separates the mount 21 from the support 22.

In particular, the intermediate wall 23 axially delimits the mount 21 towards the support 22, in particular the receptacle 22A, and/or axially delimits the support 22, in particular the receptacle 22A, towards the mount 21.

The retainer 20, in particular the intermediate wall 23, preferably comprises an in particular cross-shaped opening/aperture 24 for the plunger rod 40, preferably wherein the plunger rod 40 extends through the opening/aperture 24 of the retainer 20.

In particular, the opening/aperture 24, hereinafter referred to as aperture 24, preferably guides the plunger rod 40 radially and/or prevents or delimits a potential rotation of the plunger rod 40 relative to the retainer 20.

The administration device 100, in particular the retainer 20, preferably comprises a finger flange/collar 25, preferably wherein the finger flange/collar 25 radially extends outwards from the retainer 20.

The finger flange/collar 25, hereinafter referred to as finger flange 25, is preferably embodied as a preferably oval disk extending around mount 21, support 22, intermediate wall 23 and/or aperture 24. However, other constructional solutions are possible as well.

The securing member 90 is preferably at least essentially ring-shaped and/or surrounds/encloses the plunger rod 40 radially.

In particular, the plunger rod 40 axially extends through the securing member 90.

As already mentioned, the securing member 90 is preferably configured to secure the plunger rod 40, the first actuation part 60 and/or the second actuation part 70.

Preferably, the securing member 90 is configured to block a back-movement of the plunger rod 40 and/or the first actuation part 60. Further, the securing member 90 is preferably configured to block actuation of the second actuation part 70.

The securing member 90 preferably comprises at least one return stop 91/92, preferably wherein the return stop 91/92 is adapted to (radially) engage and/or (axially) block the first actuation part 60 and/or the plunger rod 40, at least in the initial position and/or the administration position of the plunger rod 40.

The return stop 91/92 is preferably embodied as a protrusion extending radially inwards and/or towards the plunger rod 40. Mostly preferred, the return stop 91/92 is hook-like and/or embodied as a latch.

In the present embodiment, the securing member 90 comprises a first return stop 91 and a second return stop 92, preferably wherein the first return stop 91 and the second return stop 92 are arranged on opposite sides of the securing member 90 and/or (radially) engage the plunger rod 40 from different, in particular opposite, sides.

Preferably, the securing member 90, in particular the return stop 91/92, is radially pretensioned against the plunger rod 40.

The securing member 90 is preferably compressible and/or stretchable and/or embodied as a spring.

The securing member 90 preferably comprises an (integrated) spring element 93/94, preferably wherein the spring element 93/94 is adapted to (radially) press the return stop 91/92 against the plunger rod 40.

The spring element 93/94 is preferably elastic/bendable/flexible, in particular such that the securing member 90 can be compressed or stretched.

The spring element 93/94 preferably connects the first return stop 91 and the second return stop 92.

The spring element 93/94 is preferably curved and/or arch-shaped. Mostly preferred, the spring element 93/94 is bent/curved around the plunger rod 40.

In the present embodiment, the securing member 90 comprises a first spring element 93 and a second spring element 94, preferably wherein the first spring element 93 and the second spring element 94 are arranged on opposite sides of the securing member 90 and/or around the plunger rod 40 and/or (each) connect the first return stop 91 and the second return stop 92.

As already mentioned, the securing member 90 is preferably configured to radially engage the second actuation part 70 and/or block actuation of the second actuation part 70.

The securing member 90 preferably comprises a blocking element 95, preferably wherein the blocking element 95 is configured to block rotational actuation of the second actuation part 70 and/or in a direction of actuation and/or to (radially) engage the second actuation part 70, in particular only during priming and/or only until the plunger rod 40 reaches the administration position, as will be explained later in further detail.

The blocking element 95 is preferably embodied as a protrusion/bulge extending radially outwards and/or away from the plunger rod 40 and/or towards the second actuation part 70. Preferably, the blocking element 95 radially extends through the radial opening 22C of the retainer 20, in particular the support 22, in particular such that the blocking element 95 radially engages the second actuation part 70.

The second actuation part 70 is preferably configured to axially block the plunger rod 40 and/or the first actuation part 60, in particular when the administration position is reached and/or after completing priming of the administration device 100.

In particular, the second actuation part 70 is configured to prevent that the plunger rod 40 is (directly) moved from the initial position to the final position and/or that the first actuation part 60 is completely actuated, as will be explained later in greater detail.

The second actuation part 70 is preferably at least essentially cylindrical and/or cap-like and/or embodied as a hollow cylinder.

The second actuation part 70 preferably comprises an outer part 71 and/or an inner part 72, preferably wherein the outer part 71 and/or the inner part 72 are/is at least essentially cylindrical and/or embodied as a hollow cylinder and/or wherein the inner part 72 is arranged within the outer part 71 and/or wherein the outer part 71 encloses the inner part 72.

The second actuation part 70 is preferably arranged between the container 10 and the first actuation part 60.

Preferably, the second actuation part 70 is (directly) attached, in particular axially secured, to the retainer 20, in particular the support 22, mostly preferred the wall 22B.

Preferably, the second actuation part 70, in particular its outer part 71, covers the support 22, in particular the wall 22B, and/or the securing member 90 radially and/or to the outside.

The second actuation part 70, in particular its outer part 71, preferably comprises or forms a handle/dial.

Preferably, the second actuation part 70, in particular its outer part 71, is axially attached, in particular pressed/clipped into or onto, the retainer 20, in particular the support 22, mostly preferred the wall 22B.

Preferably, the second actuation part 70, in particular the outer part 71, comprises a plurality of retaining elements 73 that radially engage and/or protrude and/or are snapped into the retainer 20, in particular the support 22, mostly preferred the wall 22B and/or a corresponding retaining element 22D, e.g. a protrusion and/or recess formed in the wall 22B, in particular such that the second actuation part 70 is axially secured to the retainer 20, in particular the support 22.

However, it is preferred that the second actuation part 70 can be moved, in particular rotated, relative to the retainer 20, preferably in a preferred/intended direction of rotation, hereinafter referred to as direction of actuation.

Preferably, the actuation mechanism, in particular the second actuation part 70, can be actuated by manually rotating the second actuation part 70 relative to the retainer 20.

In order to facilitate rotation of the second actuation part 70, the outer surface of the second actuation part 70, in particular the outer part 71, might be at least partially grooved, ripped and/or otherwise profiled.

The administration device 100, in particular the actuation mechanism and/or the second actuation part 70, preferably comprises/defines a rotation axis, preferably wherein the second actuation part 70 is rotatable about the rotation axis.

The rotation axis preferably corresponds to / coincides with the main axis A of the administration device 100. However, other solutions are possible as well, wherein the rotation axis differs from the main axis A.

It is preferred that the second actuation part 70 can be rotated only in the direction of actuation and/or in that the administration device 100, in particular the retainer 20, comprises a back-rotation lock, in particular a ratchet, for the second actuation part 70.

The direction of actuation might be indicated on the second actuation part 70 and/or the retainer 20, mostly preferred the finger flange 25, by means of a label, a mark or the like.

The second actuation part 70, in particular its outer part 71, preferably comprises or forms a back-rotation stop 74/75 and/or an actuation stop 76, preferably wherein the back-rotation stop 74/75 is configured to prevent or limit/block back-rotation of the second actuation part 70, i.e. in a direction opposite to the (intended) direction of actuation, and/or wherein the actuation stop 76 is configured to limit/block the actuation, in particular rotation, of the second actuation part 70 relative to the retainer 20, in particular in the (intended) direction of actuation.

The retainer 20, in particular the support 22, preferably comprises or forms a (corresponding) back-rotation stop 26 and/or an actuation stop 27, preferably wherein the back-rotation stop 74/75 of the second actuation part 70 and the back-rotation stop 26 of the retainer 20 interact with one another, in particular in such a manner that only a rotation of the second actuation part 70 in the (intended) direction of actuation is possible and/or such that a back-rotation of the second actuation part 70, i.e. in a direction opposite to the (intended) direction of actuation, is prevented or limited.

In particular, the back-rotation stop 26 of the retainer 20 and the back-rotation stop 74/75 of the second actuation part 70 engage one another in a form-fitting manner in a direction opposite to the (intended) direction of actuation and/or glide past each other, i.e. by elastically/temporarily deforming/bending of the back-rotation stop 74/75 of the second actuation part 70 and/or the back-rotation stop 26 of the retainer 20, in the (intended) direction of actuation.

The second actuation part 70, in particular its outer part 71, preferably comprises or forms a first back-rotation stop 74 and a second back-rotation stop 75, preferably wherein the first back-rotation stop 74 and the second back-rotation stop 75 are circumferentially spaced apart from one another and/or engage/abut the back-rotation stop 26 of the retainer 20 at different rotary positions/orientation of the second actuation part 70. Mostly preferred, the first back-rotation stop 74 engages/abuts the back-rotation stop 26 of the retainer 20 before actuating the second actuation part 70 and the second back-rotation stop 75 engages/abuts the back-rotation stop 26 of the retainer 20 after actuating the second actuation part 70, in particular in a direction opposite to the direction of actuation and/or in a form-fitting manner.

The actuation stop 76 of the second actuation part 70 and the actuation stop 27 of the retainer 20 preferably engage one another in a form-fitting manner at the end of actuation of the second actuation part 70 and/or when a predefined/final rotary orientation/position of the second actuation part 70 is reached, in particular such that further actuation of the second actuation part 70 is prevented/blocked.

The actuation stop 76 of the second actuation part 70 preferably abuts/hits the actuation stop 27 of the retainer 20 in a circumferential direction at the end of actuation of the second actuation part 70 and/or when a predefined/final rotary orientation of the second actuation part 70 is reached, in particular when reaching the releasing position, as will be explained later in further detail.

As best seen in Fig. 2, the back-rotation stop(s) 74/75 and/or the actuation stop 76 are/is preferably embodied as a preferably longitudinal protrusion, respectively, in particular a rib, protruding from the outer part 71 of the second actuation part 70 radially into the interior of the second actuation part 70, towards the support 22 and/or wall 22B of the retainer 20.

As best seen in Fig. 3, the back-rotation stop 26 and/or the actuation stop 27 are/is preferably embodied as or formed by a protrusion/bulge protruding from the support 22 and/or wall 22B radially towards the second actuation part 70, in particular the outer part 71.

As already mentioned, the inner part 72 of the second actuation part 70 is preferably embodied as a hollow cylinder arranged within and/or surrounded by the outer part 71.

The second actuation part 70, in particular the inner part 72, is preferably adapted to (radially) guide the plunger rod 40 and/or the first actuation part 60.

In particular, the second actuation part 70, mostly preferred the inner part 72, is configured to limit/block actuation of the first actuation part 60 and/or the (axial) movement of the plunger rod 40 relative to, in particular towards, the retainer 20 and/or the container 10.

The second actuation part 70, in particular the inner part 72, preferably comprises a blocking wall 77 and/or an in particular slot-shaped opening/aperture 78, preferably wherein the opening/aperture 78 is formed within the blocking wall 77.

Preferably, the plunger rod 40 and/or the first actuation part 60 extend/extends through the second actuation part 70, in particular the inner part 72, mostly preferred opening/aperture 78.

The opening/aperture 78, hereinafter referred to as opening 78, preferably guides the plunger rod 40 radially.

The shape/contour of the opening 78 preferably correspond at least essentially to the (outer) shape/contour of the plunger rod 40.

As best seen in Fig. 2, the opening 78 is preferably at least essentially slot-like.

Mostly preferred, the plunger rod 40 can only be moved/pushed through opening 78 when a predefined rotary orientation of the second actuation part 70, in particular the opening 78, relative to the plunger rod 40 and/or the retainer 20 is reached. With other words, the actuation of the first actuation part 60 and/or the movement of the plunger rod 40 depends on the rotational position of the second actuation part 70, in particular its opening 78, relative to the retainer 20, the first actuation part 60 and/or the plunger rod 40.

The plunger rod 40 is preferably adapted to (directly) act on and/or abut the stopper 18. In particular, the plunger rod 40 is adapted to push the stopper 18 towards the outlet of the container 10 and/or to push fluid 11 out of the container 10, in particular by actuating the first actuation part 60.

However, it is preferred that the stopper 18 is not rigidly attached to the plunger rod 40, in particular its tip. Preferably, the stopper 18 merely rests against and/or is loosely connected to the plunger rod 40, in particular its tip.

With other words, the plunger rod 40 and/or the stopper 18 are configured to connect merely in compression and/or when the plunger rod 40 is pushed against the stopper 18. The plunger rod 40 and the stopper 18 are preferably configured to decouple in tension and/or when the plunger rod 40 is moved/pulled out of the container 10. This prevents drawing air into the container 10, for example during a sterilizing process of the administration device 100.

In the initial state of the administration device 100 and/or in the initial position of the plunger rod 40, the stopper 18 might also be axially spaced apart from the plunger rod 40, such that the plunger rod 40 axially abuts the stopper 18 only upon actuation of the first actuation part 60.

The plunger rod 40 is preferably elongated and/or rod-shaped.

Preferably, the plunger rod 40 extends (completely) axially through the administration device 100, preferably the second actuation part 70, the securing member 90 and/or the retainer 20, mostly preferred from the first actuation part 60 through the second actuation part 70, the securing member 90 and the retainer 20 into the container 10.

The plunger rod 40 preferably comprises a front part/portion 41, an intermediate part/portion 42 and/or a rear part/portion 43, preferably wherein the intermediate part/portion 42 is (axially) arranged between the front part/portion 41 and the rear part/portion 43.

The plunger rod 40, in particular the front, intermediate and rear part/portion 41, 42, 43, is preferably integrally formed or formed as one piece.

The front part/portion 41, hereinafter referred to as front portion 41, preferably comprises or forms the (first) axial end and/or the tip of the plunger rod 40.

The front portion 41 is preferably the portion of the plunger rod 40 that at least partially extends into the container 10 and/or (directly) faces/abuts the stopper 18.

The rear part/portion 43, hereinafter referred to as rear portion 43, preferably comprises or forms a (second) axial end of the plunger rod 40. Preferably, the rear portion 43 faces away from the stopper 18.

Preferably, the first actuation part 60 is directly and/or rigidly connected to the plunger rod 40, in particular its rear portion 43. The first actuation part 60 might be formed by the rear portion 43 of the plunger rod 40 and/or integrally/as one piece with the plunger rod 40, in particular the rear portion 43.

The first actuation part 60 is preferably at least essentially plate-shaped, collar-like and/or flange-like and/or comprises an enlarged outer diameter compared to the outer diameter of the plunger rod 40, in particular its rear portion 43. Mostly preferred, the first actuation part 60 comprises or forms a thumb-rest.

By actuation, in particular depressing, of the first actuation part 60, in particular towards the second actuation part 70 and/or the retainer 20 and/or in the direction of actuation, the plunger rod 40, in particular its front portion 41, is preferably moved, in particular pushed, towards the outlet of the container 10 and/or further into the container 10.

By actuation, in particular depressing, of the first actuation part 60, in particular towards the second actuation part 70 and/or the retainer 20 and/or in the direction of actuation, the plunger rod 40, in particular its intermediate part/portion 42, is preferably moved, in particular pushed, through the second actuation part 70, the securing member 90 and/or the retainer 20.

Preferably, the direction of movement of the plunger rod 40 corresponds / coincides with the direction of actuation of the first actuation part 60 and/or is parallel to, in particular along, the main axis A.

The plunger rod 40, in particular its intermediate part/portion 42, hereinafter referred to as intermediate portion 42, is preferably adapted to mechanically interact with the actuation mechanism, the second actuation part 70 and/or the securing member 90, as will be explained later with greater detail.

The plunger rod 40, in particular the front portion 41 and/or the intermediate portion 42, preferably comprises an at least essentially cross-shaped cross section.

Preferably, the plunger rod 40, in particular the front portion 41 and/or the intermediate portion 42, comprise/comprises a cross section that varies along the main axis A.

The dimensions of the plunger rod 40, in particular the front portion 41 and/or the intermediate portion 42, are preferably smaller than the inner diameter of the container 10, in particular its main body 12, preferably such that the plunger rod 40 can move freely and/or within the container 10.

The rear portion 43 of the plunger rod 40 is preferably the portion of the plunger rod 40 that projects out of the container 10 and/or forms the transition to the first actuation part 60.

The plunger rod 40, in particular the front portion 41 and/or the intermediate portion 42, preferably comprise/comprises a plurality of, in particular four, wings/sides/rips 44-47, preferably wherein the wings/sides/rips 44-47 extend along the main axis A of the plunger rod 40 and/or project/protrude radially outwards and/or form the cross section of the plunger rod 40.

Preferably, the wings/sides/rips 44-47, hereinafter referred to as sides 44-47, are positioned/arranged/offset by at least essentially 90 degrees to one another and/or around the main axis A.

Preferably, the shape, in particular the height, of some or all sides 44-47 varies along the main axis A of the plunger rod 40 and/or in the direction of actuation.

Preferably, the plunger rod 40, in particular the front portion 41 and/or the intermediate portion 42, mostly preferred the sides 44-47, comprise/comprises indentations, projections, bulges, steps, inclinations or the like.

In the present embodiment, the plunger rod 40, in particular the front portion 41 and/or the intermediate portion 42 comprise/comprises a first side 44, a second side 45, a third side 46 and/or a fourth side 47, preferably wherein the first side 44 and the second side 45 are arranged on opposite sides of the plunger rod 40 and/or the third side 46 and the fourth side 47 are arranged on opposite sides of the plunger rod 40.

The plunger rod 40, preferably the front portion 41, preferably comprises or forms at least one guide support 44A/45A, preferably wherein the guide support 44A/45A is embodied as a bulge and/or comprises an increased height compared to the areas immediately adjacent to the guide support 44A/45A.

As best seen in Figs. 4A and 5A, the guide support 44A/45A preferably rests against and/or abuts the inner wall of the container 10, in particular its main body 12.

Preferably, the guide support 44A/45A increases the cross section of the plunger rod 40 in such a way that the plunger rod 40 (locally) fits closely and/or abuts the container 10, in particular its main body 12.

Preferably, the plunger rod 40, in particular the front portion 41, comprises a first guide support 44A and a second guide support 45A, preferably wherein the first guide support 44A and the second guide support 45A are arranged on opposite sides of the plunger rod 40 and/or are axially offset to one another, in particular such that tilting of the plunger rod 40 relative to the container 10 is prevented or at least reduced.

Mostly preferred, the first guide support 44A is formed by and/or arranged on the first side 44 and the second guide support 45A is formed by and/or arranged on the second side 45.

The plunger rod 40, in particular the intermediate portion 42, preferably comprises or forms a return stop 44B/45B, preferably wherein the return stop 44B/45B is formed by a (radial) step/protrusion of the plunger rod 40, in particular its intermediate portion 42.

The return stop 44B/45B is preferably configured to interact with (axially) abuts and/or (radially) engage the securing member 90, in particular its return stop 91/92.

In particular, the return stop 91/92 of the securing member 90 and the return stop 44B/45B of the plunger rod 40 engage one another in a form-fitting manner in a direction opposite to the (intended) direction of movement/actuation of the plunger rod 40 and/or glide past each other, e.g. by elastically/temporarily deforming/bending the securing member 90, in particular its return stop 91/92, and/or the return stop 44B/45B of the plunger rod 40, in the (intended) direction of movement/actuation, in particular such that a back-movement of the plunger rod 40 out of the container 10 and/or in a direction opposite to the direction of actuation is blocked/prevented.

The plunger rod 40, in particular the intermediate portion 42, preferably comprises a first return stop 44B and a second return stop 45B, preferably wherein the first return stop 44B and the second return stop 45B are arranged on opposite sides of the plunger rod 40, in particular the intermediate portion 42, and/or wherein the first return stop 44B and the second return stop 45B are axially offset to one another.

The first return stop 44B is preferably arranged closer to the front portion 41 and/or the tip of the plunger rod 40 than the second return stop 45B.

The first return stop 44B is preferably arranged on and/or formed by the first side 44 and the second return stop 45B is preferably arranged on and/or formed by the second side 45 of the plunger rod 40.

As already mentioned, the plunger rod 40, in particular its intermediate portion 42, is preferably moved through the securing member 90 by actuating the first actuation part 60.

Preferably, the plunger rod 40, in particular its intermediate portion 42, mechanically interacts with the securing member 90 when being moved, in particular pushed, through the securing member 90.

Preferably, the first return stop 44B of the plunger rod 40 is configured to (radially) engage the first return stop 91 of the securing member 90 and the second return stop 45B of the plunger rod 40 is configured to (radially) engage the second return stop 92 of the securing member 90.

In particular, the first return stop 44B of the plunger rod 40 (radially) engages the securing member 90, in particular its first return stop 91, in the initial position of the plunger rod 40 and the second return stop 45B of the plunger rod 40 (radially) engages the securing member 90, in particular its second return stop 92, in the administration position of the plunger rod 40.

The securing member 90, in particular its first return stop 91 (radially) engages, in particular is snapped into, the plunger rod 40, in particular the first return stop 44B, mostly preferred in the initial position of the plunger rod 40 and/or such that a back-movement of the plunger rod 40 out of the container 10 and/or in a direction opposite to the direction of actuation is blocked/prevented.

The securing member 90, in particular its second return stop 92, is preferably configured to (radially) engage, in particular snap into, the plunger rod 40, in particular the second return stop 45B, mostly preferred when the administration position of the plunger rod 40 is reached and/or such that a back-movement of the plunger rod 40 out of the container 10 and/or in a direction opposite to the direction of actuation is blocked/prevented.

The plunger rod 40, in particular the intermediate portion 42, mostly preferred the first side 44, preferably comprises or forms an inclination/ramp 44C, preferably wherein the inclination/ramp 44C, hereinafter referred to as ramp 44C, is arranged and/or starts (axially) between the first return stop 44B and the second return stop 45B and/or wherein the ramp 44C (axially) adjoins the first return stop 44B and/or (axially) extends beyond the second return stop 45B.

Preferably, the ramp 44C continuously increases the cross section of the plunger rod 40, in particular the intermediate portion 42, towards the rear portion 43/first actuation part 60. In particular, the plunger rod 40, mostly preferred the ramp 44C, is configured to stretch/widen and/or press apart the securing member 90, in particular when being moved through the securing member 90 and/or from the initial position to the administration position.

Preferably, the ramp 44C is arranged on or formed by the first side 44.

Preferably, the shape, in particular the height of the second side 45 is constant/uniform in the area opposite to the ramp 44, mostly preferred such that the cross section of the plunger rod 40 is (only) increased due to the ramp 44C.

The plunger rod 40, in particular the intermediate portion 42, preferably comprises or forms an actuation stop 46A/47A/44D/45D to control, in particular (temporarily) interrupt and/or end, actuation of the first actuation part 60 and/or movement of the plunger rod 40. Preferably, the plunger rod 40, in particular the intermediate portion 42, preferably comprises or forms a plurality of actuations stops 46A/47A and 44D/45D axially offset to one another.

The plunger rod 40, in particular the intermediate portion 42, preferably comprises or forms an actuation intermediate stop 46A/47A and/or an actuation end stop 44D/45D, preferably wherein the actuation intermediate stop 46A/47A and/or the actuation end stop 44D/45D are/is formed by a (radial) step/protrusion and/or an (sharp) edge/enlargement of the plunger rod 40, in particular the intermediate portion 42, mostly preferred its cross section.

The actuation intermediate stop 46A/47A and/or the actuation end stop 44D/45D are/is preferably configured to axially block the plunger rod 40 from being further/completely inserted into the container 10. Preferably, the actuation intermediate stop 46A/47A and/or the actuation end stop 44D/45D are/is configured to axially engage/hit/abut the second actuation part 70, in particular its inner part 72, mostly preferred the blocking wall 77, in pre-defined positions of the plunger rod 40 relative to the second actuation part 70, mostly preferred in the administration position and/or in the final position.

The actuation intermediate stop 46A/47A is preferably arranged axially in front of the actuation end stop 44D/45D (in the direction of actuation/movement) and/or between the second return stop 45B and the actuation end stop 44D/45D.

With other words, the actuation intermediate stop 46A/47A preferably (axially) engages the second actuation part 70, in particular its blocking wall 77, before the actuation end stop 44D/45D when actuating the first actuation part 60.

Preferably, the third side 46 and/or the fourth side 47 of the plunger rod 40 comprise/comprises or form/forms the actuation intermediate stop 46A/47A.

In the present embodiment, the plunger rod 40, in particular the intermediate portion 42, comprises two actuation intermediate stops 46A and 47A arranged on opposite sides of the plunger rod 40, preferably wherein the third side 46 comprises or forms the first actuation intermediate stop 46A and the fourth side 47 comprises or forms the second actuation intermediate stop 47A.

Preferably, the first side 44 and/or the second side 45 of the plunger rod 40 comprise/comprises or form/forms the actuation end stop 44D/45D.

In the present embodiment, the plunger rod 40, in particular the intermediate portion 42, comprises two actuation end stops 44D and 45D arranged on opposite sides, preferably wherein the first side 44 comprises or forms the first actuation end stop 44D and the second side 45 comprises or forms the second actuation end stop 45D.

In the following, different states of the administration device 100 and the interaction between the parts/components of the administration device 100 during use will be explained with reference to Figs. 4A-4D, 5A-5D, 6A-6B, 7A-7B and 8A 8B.

Fig. 4A-4D show a section of the administration device 100 in different positions/states, respectively. Figs. 5A-5D correspond to Figs. 4A-4D and show the administration device 100 in a section which is rotated by 90° with respect to the corresponding Figs. 4A-4D.

Figs. 4A and 5A show the administration device 100 in the initial position/state, i.e. before actuating the first actuation part 60 for the first time. Figs. 4B and 5B show the administration device 100 in the administration position/state, i.e. after (partially) actuating the first actuation part 60 for the first time and before administering the fluid 11, the second actuation part 70 being (still) unactuated and blocking the first actuation part 60 from being further/completely actuated. Figs. 4C and 5C show the administration device 100 in the administration position/state after actuating the second actuation part 70 such that the first actuation part 60 can be actuated for the second time. Figs. 4D and 5D show the administration device 100 in the final position/state in which the first actuation part 60 has been completely actuated, i.e. after administering the fluid 11.

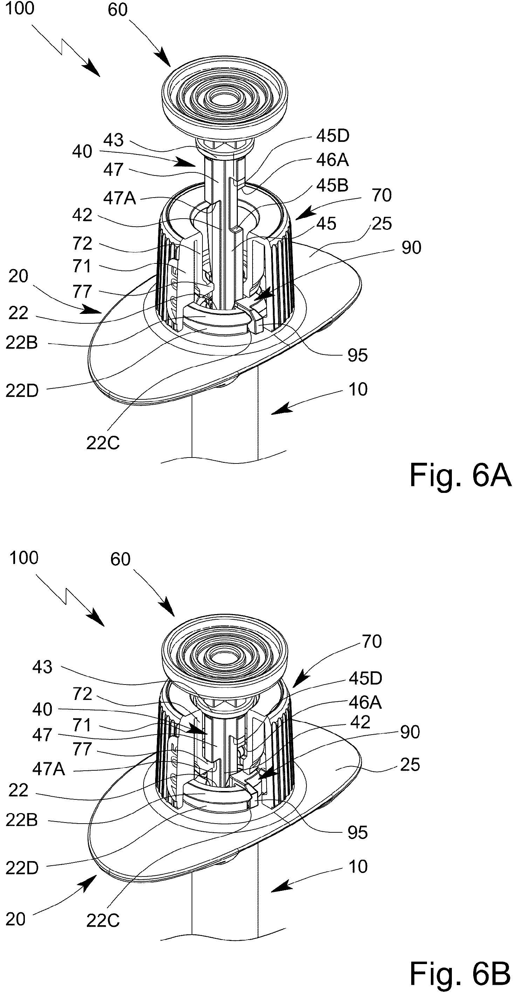

Fig. 6A shows the administration device 100 in the initial position/state, i.e. before actuating the first actuation part 60 for the first time, the securing member 90 being in the engaging position and preventing actuation of the second actuation part 70. Fig. 6B shows the administration device 100 in the administration position/state, i.e. after (partially) actuating the first actuation part 60, the securing member 90 being in the disengaging position such that the second actuation part 70 can be actuated.

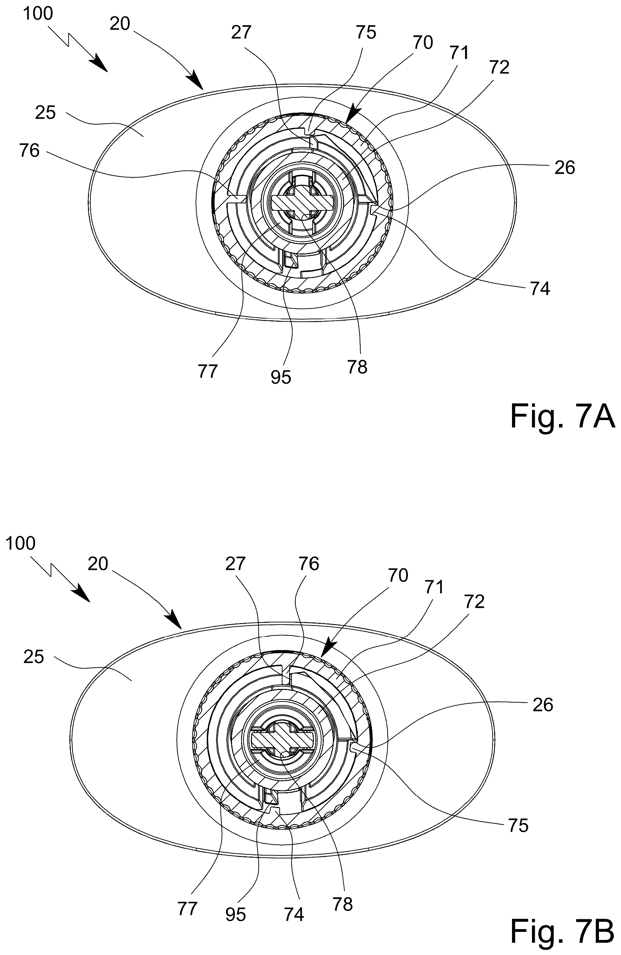

Fig. 7A is a schematic cross section of the administration device 100 in the administration position/state, i.e. after (partially) actuating the first actuation part 60, the second actuation part 70 being (still) unactuated. Fig. 7B is a schematic cross section of the administration device 100 in the administration position/state after actuating the second actuation part 70.

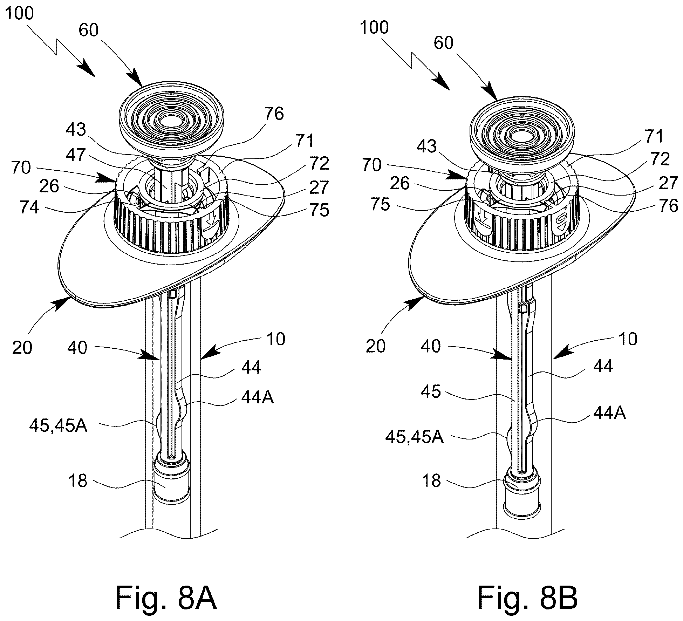

Fig. 8A is a perspective partial section of the administration device 100 in the administration position/state according to Fig. 7A. Fig. 8B is a perspective partial section of the administration device 100 in the administration position/state according to Fig. 7B.

The initial position is preferably the (axial) position of the plunger rod 40 and/or the stopper 18 relative to the retainer 20 and/or container 10 before actuating the first actuation part 60 and/or the second actuation part 70 and/or before priming of the administration device 100 and/or in the initial state of the administration device 100.

In the initial state of the administration device 100 and/or when the plunger rod 40 and/or the stopper 18 are/is in the initial position, the first actuation part 60 and the second actuation part 70 are preferably unactuated.

In the initial position/state, the plunger rod 40, in particular its rear portion 43, axially protrudes out of the retainer 20 and/or the second actuation part 70.

It is preferably not possible to (directly) actuate the second actuation part 70 and/or to (directly) administer the fluid 11 in the initial state of the administration device 100 and/or when the plunger rod 40 and/or the stopper 18 are/is in the initial position.

Preferably, only the first actuation part 60 is (partially) actuatable in the initial state/position. The second actuation part 70 is preferably not actuatable in the initial state/position.

In the initial position/state, the second actuation part 70 is preferably blocked rotationally and/or in the direction of actuation, mostly preferred by means of the securing member 90.

However, other solutions are possible as well, wherein it is not possible to (directly) actuate the first actuation part 60 and/or wherein only the second actuation part 70 is (partially) actuatable in the initial state/position. In particular, the second actuation part 70 can be configured to axially block the plunger rod 40 and/or the first actuation part 60 in the initial position/state and/or is preferably to be actuated, in particular (partially) turned/rotated relative to the retainer 20 and/or around the plunger rod 40, in order to release the plunger rod 40 and/or such that the first actuation part 60 can be actuated, in particular for the first time.

As best seen in Fig. 4A, the plunger rod 40 is preferably axially secured from being pulled out in the initial state/position, preferably by means of the securing member 90.

In particular, the securing member 90 is configured to block a back-movement of the plunger rod 40 and/or the first actuation part 60 in the initial state/position.

Preferably, the securing member 90, in particular its first return stop 91, (axially) abuts and/or (radially) engages the plunger rod 40, in particular the first return stop 44B of the plunger rod 40, in the initial state/position, mostly preferred such that a back-movement of the plunger rod 40 and/or a movement of the plunger rod 40 out of the container 10 is blocked.

As already mentioned, it is preferably not possible to (directly) actuate the second actuation part 70 in the initial state/position, during actuating the first actuation part 60 and/or during movement of the plunger rod 40 and/or the stopper 18 from the initial position to the administration position.

Preferably, the second actuation part 70 is blocked, in particular rotationally and/or in the direction of actuation, in the initial state/position, during actuating the first actuation part 60 and/or during movement of the plunger rod 40 and/or the stopper 18 from the initial position to the administration position and/or before reaching the administration position of the plunger rod 40, mostly preferred by means of the securing member 90, in particular its blocking element 95.

As best seen in Fig. 6A, the securing member 90, in particular the blocking element 95, preferably (radially) engages the second actuation part 70 in the initial state/position, during actuating the first actuation part 60 and/or during movement of the plunger rod 40 and/or the stopper 18 from the initial position to the administration position and/or before reaching the administration position.

Preferably, the securing member 90, in particular its blocking element 95, extends through the wall 22B and/or the radial opening 22C of the support 22 and/or protrudes out of the wall 22B and/or the radial opening 22C of the support 22, in particular such that the second actuation part 70 is blocked from being actuated/rotated and/or in the direction of actuation/rotation.

As best seen in Fig. 7A, the second actuation part 70 is preferably (also) blocked in a direction opposite to the direction of actuation in the initial state/position.

Preferably, the retainer 20, in particular the back-rotation stop 26, (circumferentially) abuts/blocks and/or (radially) engages the second actuation part 70, in particular the first rotation stop 74 thereof, in a direction opposite to the direction of actuation and/or such that back-rotation is prevented in the initial state/position.

It is thus preferred that the position and/or angle of rotation of the second actuation part 70 is at least essentially fixed or limited, in particular in the direction of actuation and in the direction opposite to the direction of actuation, - in the initial state/position and/or during movement of the stopper 18 and/or the plunger rod 40 from the initial position to the administration position - and/or before reaching the administration position by means of the securing member 90, in particular its blocking element 95, and by means of the retainer 20, in particular its back-rotation stop 26.

The first actuation part 60 can be manually actuated in order to axially move the plunger rod 40 relative to the retainer 20 and/or the container 10, in particular from the initial position to the administration position in a first step and/or for priming of the administration device 100 and/or - in particular subsequently and/or after actuating the second actuation part 70 - from the administration position to the final position in a second step and/or for administering the fluid 11, mostly preferred by pushing, in particular depressing, the first actuation part 60 towards the second actuation part 70 and/or the retainer 20.

By actuating, in particular depressing, the first actuation part 60, the stopper 18 and/or the plunger rod 40 can be moved, in particular pushed, out of the initial position, in particular from the initial position to the administration position, and/or - in particular subsequently and/or after actuating the second actuation part 70 - from the administration position to the final position.

Figs. 4B, 5B und 6B show the administration device 100 in the administration position after (partially) actuating the first actuation part 60 for the first time and before actuating the second actuation part 70.

By actuating, in particular depressing, the first actuation part 60, the plunger rod 40, in particular the intermediate portion 42, mostly preferred its ramp 44C, is moved, in particular pushed, through the securing member 90, thereby (further) tensioning/widening the securing member 90 and/or pressing the first return stop 91 radially outwards and/or pressing apart the first return stop 91 and the second return stop 92, in particular such that the securing member 90 is (further) pretensioned against the plunger rod 40 during movement of the plunger rod 40 from the initial position towards the administration position.

The plunger rod 40, in particular the second side 45, comprises an indentation 45C, preferably wherein the indentation 45C forms the second return stop 45B.

When reaching the administration position and/or at the end of the priming process and/or when the indentation 45C and/or the second return stop 45B is pushed/arranged directly adjacent to and/or at the same (axial) level as the securing member 90, the (tensioned) securing member 90 can/is at least partially, preferably completely, relax/released and/or the securing member 90, in particular its second return stop 92, (automatically) engages and/or snaps into the plunger rod 40, in particular its second return stop 45B and/or the indentation 45C.

In this way, the securing member 90, in particular its second return stop 92, preferably (radially) engages the plunger rod 40, in particular its second return stop 45B and/or indentation 45C, preferably automatically and/or by spring force, mostly preferred such that a back-movement of the plunger rod 40 and/or a movement of the plunger rod 40 out of the container 10 is prevented/blocked in the administration position.

Additionally or alternatively, the securing member 90, in particular its blocking element 95, (radially) disengages and/or (rotationally) releases the second actuation part 70, preferably automatically and/or by spring force, mostly preferred such that the second actuation part 70 can be actuated, in particular for the first time.

Thus, when reaching the administration position, at the end of the priming process and/or when the indentation 45C and/or the second return stop 45B is pushed directly adjacent to the securing member 90, the securing member 90, in particular its second return stop 92, (automatically and/or audibly) engages and/or snaps into the plunger rod 40, in particular its second return stop 45B and/or the indentation 45C, in particular such that the securing member 90 (radially) engages and/or (axially) blocks/secures the plunger rod 40 (in a direction opposite to the direction of actuation) and, further, (radially) disengages and/or (rotationally) releases the second actuation part 70.

With other words, by actuating the first actuation part 60 for the first time and/or during movement of the plunger rod 40 from the initial position to the administration position, the securing member 90, in particular its spring elements 93/94, is first tensioned and subsequently and/or when reaching the administration position - in particular suddenly/abruptly and/or audibly - at least partially, preferably completely, released/relaxed.

In this way, the securing member 90 is (automatically) moved from the engaging position to the disengaging position, the second actuation part 70 is (automatically) released/unblocked and/or the plunger rod 40 is (automatically) secured (in the direction opposite to its direction of actuation/movement).

Preferably, an audible/haptic feedback is provided that clearly indicates the end of priming and/or when the administration position is reached.

The administration position is preferably the (axial) position of the stopper 18 and/or the plunger rod 40 relative to the retainer 20 and/or the container 10 after completing the priming process and/or after actuating, in particular depressing, the first actuation part 60, in particular partially and/or for the first time.

As already mentioned, the second actuation part 70 is preferably configured to axially block the plunger rod 40 and/or the first actuation part 60 (in the direction of actuation) and/or to prevent complete actuation of the first actuation part 60 and/or complete insertion of the plunger rod 40 into the container 10, in particular when the administration position is reached.

The first actuation part 60 can only be (initially) actuated and/or the plunger rod 40 can only be (initially) moved until the second actuation part 70 axially blocks the first actuation part 60 and/or the plunger rod 40. With other words, the first actuation part 60 can only be partially actuated due to the (blocking of the) second actuation part 70.

Further actuation of the first actuation part 60 and/or movement of the plunger rod 40 from the administration position to the final position is thus prevented/blocked by means of the second actuation part 70, at least when the second actuation part 70 is unactuated and/or in the blocking position. Only after actuating the second actuation part 70 and/or moving the second actuation part 70 from the blocking position to the releasing position, the first actuation part 60 can be actuated again and/or for a second time and/or the plunger rod 40 can be moved from the administration position to the final position.

As best seen in Fig. 5B, the plunger rod 40, in particular the actuation intermediate stop 46A/47A, abuts the second actuation part 70, in particular the blocking wall 77, in the administration position. Thus, when reaching the administration position, (further) actuation of the first actuation part 60 is (temporarily) blocked/prevented by means of the second actuation part 70.

Preferably, the second actuation part 70 is to be actuated, in particular turned/rotated or slided relative to the retainer 20 and/or around the plunger rod 40, preferably by at least essentially 90 degrees, in order to (axially) release the plunger rod 40 and/or the first actuation part 60 and/or such that the first actuation part 60 can be actuated, in particular completely, again, for the second time and/or for administering the fluid 11.

As best seen comparing Figs. 5B and 5C, the second actuation part 70 is to be actuated - in particular by sliding or rotating - to align the second actuation part 70, in particular the opening 78, with and/or relative to the first actuation part 60 and/or the plunger rod 40, in particular the actuation intermediate stop 46A/47A, and/or such that the plunger rod 40, in particular the actuation intermediate stop 46A/47A, can move through the opening 78 of the second actuation part 70.

Preferably, the second actuation part 70 can (only) be actuated until a predefined position is reached and/or until further actuation, in particular rotation, of the second actuation part 70 is blocked by the retainer 20, in particular the actuation stop 27, and/or until the actuation stop 76 of the second actuation part 70 hits/abuts the actuation stop 27 of the retainer 20.

As best seen in Fig. 7B, the position and/or angle of rotation of the second actuation part 70 is at least essentially fixed and/or limited after actuating the second actuation part 70.

Preferably, further actuation/rotation is blocked/prevented by means of the actuation stop 27 and back-rotation is blocked/prevented by means of the back-rotation stop 26 of the retainer 20.

After (completely) actuating the second actuation part 70, the retainer 20, in particular the support 22, mostly preferred the back-rotation stop 26, (circumferentially) abuts/blocks and/or (radially) engages the second actuation part 70, in particular the second back-rotation stop 75, in a direction opposite to the direction of actuation/rotation and the retainer 20, in particular the support 22, mostly preferred the actuation stop 27, engages the second actuation part 70, in particular the actuation stop 76, in the direction of actuation/rotation.

Optionally, the retainer 20 provides an increased rotational resistance at the beginning and/or at the end of actuating the second actuation part 70, thereby providing an audible/haptic feedback to a user/practitioner indicating the beginning and/or the end of actuation of the second actuation part 70.

In the present embodiment, the second actuation part 70, in particular the second back-rotation stop 75, is to be pushed over a (radial) protrusion at the beginning and at the end of the actuation of the second actuation part 70. However, other solutions are possible as well.

After actuating the second actuation part 70, the first actuation part 60 is (axially) unblocked/released (in its direction of actuation) and can be actuated, in particular again and/or for the second time and/or for moving the plunger rod 40 from the administration position to the final position and/or further into the container 10.

By actuating, in particular depressing, the first actuation part 60, in particular again and/or for a second time and/or after actuating the second actuation part 70, the administration of the fluid 11 can be carried out and/or the plunger rod 40 and/or the stopper 18 are/is moved to the final position and/or further into the container 10.

Figs. 4D and 5D show the administration device 100 in the final position/state, i.e. after completely actuating the first actuation part 60.

As best seen comparing Fig. 4C and 4D, the fist actuation part 60 can be actuated, in particular depressed, preferably only until the plunger rod 40, in particular the actuation stop 44D/45D, (axially) abuts/engages the second actuation part 70, in particular the blocking wall 77. Alternatively or additionally, the first actuation part 60 may abut/hit the actuation part 70, in particular its outer part 71.

Thus, in the final position/state, the plunger rod 40 and/or the first actuation part 60 (axially) abuts/engages the second actuation part 70, in particular its blocking wall 77, thereby in particular preventing further actuation of the first actuation part 60 and/or insertion of the plunger rod 40.

In particular, the position of the plunger rod 40 and/or the stopper 18 is predefined by means of the second actuation part 70, in particular its blocking wall 77, in the administration position and in the final position, thereby increasing the dose accuracy.

The final position is preferably the (axial) position of the stopper 18 and/or plunger rod 40 relative to the retainer 20 and/or container 10 immediately (after) administration and/or after (completely) actuating the first actuation part 60 and/or in the final state of the administration device 100.

In the final state of the administration device 100 and/or when the plunger rod 40 and/or the stopper 18 are/is in the final position, the administration of the fluid 11 has been carried out, the first actuation part 60 is completely actuated and/or further actuation of the first actuation part 60 and/or the second actuation part 70 is in particular mechanically prevented.

As already mentioned, the administration device 100 may be adapted for ophthalmic injections. The administration device 100 preferably comprises suitably small volumes of the fluid 11 to be injected.

The administration device 100, in particular the container 10, may be silicon-free, or at least essentially silicon-free or may comprise a low level of silicon as lubricant.

Preferably, the administration device 100 and/or the container 10 meets the requirements of USP 789.

The administration device 100, in particular the container 10, may be filled with any suitable injectable fluid/liquid/medication 11.

According to a preferred embodiment, the administration device 100, in particular the container 10, is filled with the fluid 11, in particular an injectable medicament, comprising an active suitable for the treatment of an ocular disease. Examples for such ocular diseases include choroidal neovascularisation, age-related macular degeneration (AMD, both wet and dry forms), macular edema secondary to retinal vein occlusion (RVO) including both branch RVO (bRVO) and central RVO (cRVO), choroidal neovascularisation secondary to pathologic myopia (PM), diabetic macular edema (DME), diabetic macular ischemia (DMI), geographic atrophy, diabetic retinopathy, and proliferative retinopathy.

According to another preferred embodiment, the fluid 11, in particular the injectable medicament, comprises a biologic active ingredient, such as an antibody (or a fragment thereof) or a non-antibody protein.

In one embodiment, the fluid 11, in particular the injectable medicament, comprises a VEGF antagonist, a multi-specific anti-VEGF/Ang2 antibody or a fragment thereof, a multi-specific anti-VEGF/AngPT2 antibody or a fragment thereof, a multi-specific anti-VEGF/TrkB agonist, or a multi-specific anti-VEGF/APL-2 complement C3 inhibitor, such as ranibizumab, bevacizumab, aflibercept, brolucizumab, conbercept (KH902) and the related glycoform KH906, faricinab or pazopanib.

Individual aspects and features of the present invention may be implemented independently from one another, but also in any combination and/or order.

In particular, the present invention relates also to any of the following aspects which can be realized independently or in any combination, also in combination with any aspect described above:
1. Administration device (100) for in particular intravitreal administration of a fluid (11), the administration device (100) comprising:
   a retainer (20) for a container (10) containing the fluid (11), and
   an actuation mechanism comprising a plunger rod (40), a first actuation part (60) and a second actuation part (70), the second actuation part (70) being movably attached to the retainer (20),
   wherein the plunger rod (40) is adapted to act on a stopper (18) movably arranged within the container (10), and
   wherein the first actuation part (60) can be manually actuated in order to axially move the plunger rod (40) relative to the retainer (20),
   characterized,
   in that the second actuation part (70) is configured to axially block the plunger rod (40) and/or the first actuation part (60), wherein the second actuation part (70) is to be actuated in order to axially release the plunger rod (40) and/or the first actuation part (60) such that the first actuation part (60) can be actuated, and/or
   in that the actuation mechanism comprises a securing member (90), wherein the securing member (90) is configured to block a back-movement of the plunger rod (40) and/or the first actuation part (60) at least in an initial position and/or in an administration position of the plunger rod (40) and wherein the securing member (90) is configured to block the second actuation part (70) in the initial position and/or before reaching the administration position.
2. Administration device, preferably according to aspect 1, characterized in that the plunger rod (40) axially extends through the retainer (20) and/or the second actuation part (70).
3. Administration device, preferably according to aspect 1 or 2, characterized in that the first actuation part (60) can be actuated by - in particular manually - pushing the first actuation part (60) towards the second actuation part (70) and/or towards the retainer (20).
4. Administration device, preferably according to any of the preceding aspects, characterized in that the second actuation part (70) can be actuated by - in particular manually -rotating or sliding the second actuation part (70) relative to the retainer (20) and/or around the plunger rod (40), preferably by at least essentially 90 degrees and/or in order to change the alignment of the second actuation part (70) relative to the first actuation part (60) and/or the plunger rod (40).
5. Administration device, preferably according to any of the preceding aspects, characterized in that the plunger rod (40) comprises a cross section that varies along the main axis (A) of the plunger rod (40) and/or in that the second actuation part (70) comprises an opening (78), wherein the second actuation part (70) is to be actuated to - in particular rotationally or slidingly - align the opening (78) with the plunger rod (40) such that the plunger rod (40) can move through the opening (78).
6. Administration device, preferably according to any of the preceding aspects, characterized in that the first actuation part (60) can be - in particular manually - actuated in order to move the plunger rod (40) from an initial position to an administration position and/or - in particular subsequently and/or after actuating the second actuation part (70) - from the administration position to a final position.
7. Administration device, preferably according to any of the preceding aspects, characterized in that the second actuation part (70) is configured to axially block the plunger rod (40) and/or the first actuation part (60) in the initial position of the plunger rod (40), wherein the second actuation part (70) is to be actuated, in particular rotated or slided relative to the retainer (20) and/or around the plunger rod (40), preferably by at least essentially 90 degrees, in order to unblock the plunger rod (40) and/or the first actuation part (60) and/or such that the first actuation part (60) can be actuated, in particular for the first time and/or for priming of the administration device (100), and/or such that the plunger rod (40) can be moved from the initial position to the administration position.
8. Administration device, preferably according to any of the preceding aspects, characterized in that the second actuation part (70) is configured to axially block the plunger rod (40) and/or the first actuation part (60) in the administration position of the plunger rod (40), wherein the second actuation part (70) is to be actuated, in particular rotated or slided relative to the retainer (20) and/or around the plunger rod (40), preferably by at least essentially 90 degrees, in order to unblock the plunger rod (40) and/or the first actuation part (60) and/or such that the first actuation part (60) can be actuated, in particular for the second time and/or for administrating of the fluid (11), and/or such that the plunger rod (40) can be moved from the administration position to the final position.
9. Administration device, preferably according to any of the preceding aspects, characterized in that the actuation mechanism comprises a securing member (90) for securing the plunger rod (40), the first actuation part (60) and/or the second actuation part (70), preferably wherein the retainer (20) comprises a receptacle (22A) for the securing member (90).
10. Administration device, preferably according to aspect 9, characterized in that the securing member (90) is ring-shaped and/or in that the plunger rod (40) axially extends through the securing member (90).
11. Administration device, preferably according to aspect 9 or 10, characterized in that the securing member (90) is embodied as a preferably flexible ring and/or in that the securing member (90) is coupled loosely and/or with radial play to the plunger rod (40) in the initial position and/or in the administration position and/or in that the securing member (90) is radially pretensioned against and/or tightly coupled to the plunger rod (40) during movement of the plunger rod (40) from the initial position towards the administration position.
12. Administration device, preferably according to any of the aspects 9 to 11, characterized in that the securing member (90) engages the plunger rod (40) and/or in that the securing member (90) is configured to block a back-movement of the plunger rod (40) and/or the first actuation part (60), at least in the initial position and/or in the administration position of the plunger rod (40).
13. Administration device, preferably according to any of the aspects 9 to 12, characterized in that the securing member (90) engages the second actuation part (70) and/or in that the securing member (90) is configured to block actuating the second actuation part (70), in particular in the initial position and/or before and/or only until the plunger rod (40) reaches the administration position, and/or to automatically unblock actuation of the second actuation part (70) upon actuating the first actuation part (60) and/or when the administration position is reached.
14. Administration device, preferably according to any of the aspects 9 to 13, characterized in that the plunger rod (40) comprises or forms a ramp (44C) for the securing member (90) and/or in that the plunger rod (40), in particular its ramp (44C), is configured to - in particular further - tension and/or widen the securing member (90) upon actuating the first actuation part (60) and/or during movement of the plunger rod (40) from the initial position towards the administration position.
15. Administration device, preferably according to any of the aspects 9 to 14, characterized in that the plunger rod (40) comprises an indentation (45C) for the securing member (90) and/or in that the plunger rod (40), in particular its indentation (45C), is configured to at least partially, preferably completely, and/or audibly release the securing member (90) and/or to disengage the securing member (90) from the second actuation part (70) upon actuating the first actuation part (60) and/or when reaching the administration position, in particular such that the second actuation part (70) can be actuated, in particular for the first time, and/or in such a way that an audible and/or haptic feedback is provided.
16. Administration device (100) for in particular intravitreal administration of a fluid (11), the administration device (100) comprising:
   a retainer (20) for holding a container (10) containing the fluid (11), and
   an actuation mechanism comprising a plunger rod (40), a first actuation part (60) and a second actuation part (70), the second actuation part (70) being movably attached to the retainer (20),
   wherein the plunger rod (40) is adapted to act on a stopper (18) movably arranged within the container (10), and
   wherein the first actuation part (60) can be manually actuated in order to axially move the plunger rod (40) relative to the retainer (20) from an initial position to an administration position and from the administration position to a final position,
   **characterized,**
   in that the second actuation part (70) is configured to axially block the plunger rod (40) and/or the first actuation part (60) in the administration position of the plunger rod (40), wherein the second actuation part (70) is to be actuated in order to axially release the plunger rod (40) and/or the first actuation part (60) such that the first actuation part (60) can be actuated and the plunger rod (40) can be moved from the administration position to the final position, and/or
   in that the actuation mechanism comprises a securing member (90), wherein the securing member (90) is configured to block a back-movement of the plunger rod (40) and/or the first actuation part (60) at least in the initial position and/or in the administration position of the plunger rod (40) and wherein the securing member (90) is configured to block the second actuation part (70) in the initial position and/or before reaching the administration position and to automatically unblock actuation of the second actuation part (70) upon actuating the first actuation part (60) and/or when the administration position is reached.
17. Administration device according to aspect 16, characterized in that the plunger rod (40) axially extends through the retainer (20) and/or the second actuation part (70).
18. Administration device according to aspect 16 or 17, characterized in that the first actuation part (60) can be actuated by - in particular manually - pushing the first actuation part (60) towards the second actuation part (70) and/or towards the retainer (20).
19. Administration device according to any of the aspects 16 to 18, characterized in that the second actuation part (70) can be actuated by - in particular manually - rotating or sliding the second actuation part (70) relative to the retainer (20) and/or around the plunger rod (40), preferably by at least essentially 90 degrees and/or in order to change the alignment of the second actuation part (70) relative to the first actuation part (60) and/or the plunger rod (40).
20. Administration device according to any of the aspects 16 to 19, characterized in that the plunger rod (40) comprises a cross section that varies along the main axis (A) of the plunger rod (40).
21. Administration device according to any of the aspects 16 to 20, characterized in that the second actuation part (70) comprises an opening (78), wherein the second actuation part (70) is to be actuated to - in particular rotationally or slidingly - align the opening (78) with the plunger rod (40) such that the plunger rod (40) can move through the opening (78).
22. Administration device according to any of the aspects 16 to 21, characterized in that the first actuation part (60) can be - in particular manually - actuated in order to move the plunger rod (40) from the initial position to the administration position and subsequently and/or after actuating the second actuation part (70) from the administration position to the final position.
23. Administration device according to any of the aspects 16 to 22, characterized in that the first actuation part (60) is to be actuated before actuating the second actuation part (70) - in particular for the first time - in order to move the plunger rod (40) from the initial position to the administration position and/or to unblock the second actuation part (70) and/or in that the first actuation part (60) is to be actuated again and/or after actuating the second actuation part (70) in order to move the plunger rod (40) from the administration position to the final position.
24. Administration device according to any of the aspects 16 to 23, characterized in that the second actuation part (70) is blocked in the initial position and/or before reaching the administration position, in particular by means of the securing member (90), and/or in that the second actuation part (70) can be only actuated, in particular for the first time, upon actuation of the first actuation part (60).
25. Administration device according to any of the aspects 16 to 22, characterized in that the second actuation part (70) is configured to axially block the plunger rod (40) and/or the first actuation part (60) in the initial position of the plunger rod (40), wherein the second actuation part (70) is to be actuated, in particular rotated or slided relative to the retainer (20) and/or around the plunger rod (40), preferably by at least essentially 90 degrees and/or for the first time, in order to unblock the plunger rod (40) and/or the first actuation part (60) and/or such that the first actuation part (60) can be actuated, in particular for the first time and/or for priming of the administration device (100), and/or such that the plunger rod (40) can be moved from the initial position to the administration position.
26. Administration device according to any of the aspects 16 to 25, characterized in that the second actuation part (70) is configured to axially block the plunger rod (40) and/or the first actuation part (60) in the administration position of the plunger rod (40), wherein the second actuation part (70) is to be actuated, in particular rotated or slided relative to the retainer (20) and/or around the plunger rod (40), preferably by at least essentially 90 degrees and/or for the second time, in order to unblock the plunger rod (40) and/or the first actuation part (60) and/or such that the first actuation part (60) can be actuated, in particular for the second time and/or for administrating of the fluid (11), and/or such that the plunger rod (40) can be moved from the administration position to the final position.
27. Administration device according to any of the aspects 16 to 26, characterized in that the actuation mechanism comprises a securing member (90) for securing the plunger rod (40), the first actuation part (60) and/or the second actuation part (70), preferably wherein the retainer (20) comprises a receptacle (22A) for the securing member (90).
28. Administration device according to aspect 27, characterized in that the securing member (90) is ring-shaped and/or in that the plunger rod (40) axially extends through the securing member (90).
29. Administration device according to aspect 27 or 28, characterized in that the securing member (90) is embodied as a preferably flexible ring.
30. Administration device according to any of the aspects 27 to 29, characterized in that the securing member (90) is coupled loosely and/or with radial play to the plunger rod (40) in the initial position and/or in the administration position.
31. Administration device according to any of the aspects 27 to 30, characterized in that the securing member (90) is radially pretensioned against and/or tightly coupled to the plunger rod (40) during movement of the plunger rod (40) from the initial position towards the administration position.
32. Administration device according to any of the aspects 27 to 31, characterized in that the securing member (90) engages the plunger rod (40) and/or in that the securing member (90) is configured to block a back-movement of the plunger rod (40) and/or the first actuation part (60), at least in the initial position and/or in the administration position of the plunger rod (40).
33. Administration device according to any of the aspects 27 to 32, characterized in that the securing member (90) engages the second actuation part (70) and/or in that the securing member (90) is configured to block actuating the second actuation part (70), in particular in the initial position and/or before and/or only until the plunger rod (40) reaches the administration position, and/or to automatically unblock actuation of the second actuation part (70) upon actuating the first actuation part (60) and/or when the administration position is reached.
34. Administration device according to any of the aspects 27 to 33, characterized in that the plunger rod (40) comprises or forms a ramp (44C) for the securing member (90).
35. Administration device according to any of the aspects 27 to 34, characterized in that the plunger rod (40), in particular a ramp (44C) thereof, is configured to - in particular further - tension and/or widen the securing member (90) upon actuating the first actuation part (60) and/or during movement of the plunger rod (40) from the initial position towards the administration position.
36. Administration device according to any of the aspects 27 to 35, characterized in that the plunger rod (40) comprises an indentation (45C) for the securing member (90).
37. Administration device according to any of the aspects 27 to 36, characterized in that the plunger rod (40), in particular an indentation (45C) thereof, is configured to at least partially, preferably completely, and/or audibly release the securing member (90) and/or to disengage the securing member (90) from the second actuation part (70) upon actuating the first actuation part (60) and/or when reaching the administration position, in particular such that the second actuation part (70) can be actuated, in particular for the first time, and/or in such a way that an audible and/or haptic feedback is provided.

### List of reference signs:

- 100: administration device

- 10: container
- 11: fluid
- 12: main body
- 13: first axial end
- 14: second axial end
- 15: lock
- 16: connector
- 17: cap
- 18: stopper
- 19: needle

- 20: retainer

- 21: mount
- 21A: radial opening
- 21B: axial opening

- 22: support
- 22A: receptacle
- 22B: wall
- 22C: radial opening
- 22D: retaining element

- 23: intermediate wall
- 24: aperture
- 25: finger flange
- 26: back-rotation stop
- 27: actuation stop

- 40: plunger rod
- 41: front portion
- 42: intermediate portion
- 43: rear portion

- 44: first side
- 44A: first guide support
- 44B: first return stop
- 44C: ramp
- 44D: actuation end stop

- 45: second side
- 45A: second guide support
- 45B: second return stop
- 45C: indentation
- 45D: actuation end stop

- 46: third side
- 46A: actuation intermediate stop

- 47: fourth side
- 47A: actuation intermediate stop

- 60: first actuation part

- 70: second actuation part
- 71: outer part
- 72: inner part
- 73: retaining element
- 74: first back-rotation stop
- 75: second back-rotation stop
- 76: actuation stop
- 77: blocking wall
- 78: opening

- 90: securing member
- 91: first return stop
- 92: second return stop
- 93: first spring element
- 94: second spring element
- 95: blocking element

- A: main axis

## Claims

1. Administration device (100) for in particular intravitreal administration of a fluid (11), the administration device (100) comprising:
a retainer (20) for holding a container (10) containing the fluid (11), and
an actuation mechanism comprising a plunger rod (40), a first actuation part (60) and a second actuation part (70), the second actuation part (70) being movably attached to the retainer (20),
wherein the plunger rod (40) is adapted to act on a stopper (18) movably arranged within the container (10), and
wherein the first actuation part (60) can be manually actuated in order to axially move the plunger rod (40) relative to the retainer (20) from an initial position to an administration position and from the administration position to a final position,
**characterized,**
**in that** the second actuation part (70) is configured to axially block the plunger rod (40) and/or the first actuation part (60) in the administration position of the plunger rod (40), wherein the second actuation part (70) is to be actuated in order to axially release the plunger rod (40) and/or the first actuation part (60) such that the first actuation part (60) can be actuated and the plunger rod (40) can be moved from the administration position to the final position.

2. Administration device according to claim 1, **characterized in that** the plunger rod (40) axially extends through the retainer (20) and/or the second actuation part (70).

3. Administration device according to claim 1 or 2, **characterized in that** the first actuation part (60) can be actuated by - in particular manually - pushing the first actuation part (60) towards the second actuation part (70) and/or towards the retainer (20).

4. Administration device according to any of the preceding claims, **characterized in that** the second actuation part (70) can be actuated by manually sliding the second actuation part (70) relative to the retainer (20), preferably in order to change the alignment of the second actuation part (70) relative to the first actuation part (60) and/or the plunger rod (40).

5. Administration device according to any of the preceding claims, **characterized in that** the plunger rod (40) comprises an at least essentially cross-shaped cross section and/or a cross section that varies along the main axis (A) of the plunger rod (40).

6. Administration device according to any of the preceding claims, **characterized in that** the plunger rod (40) comprises a plurality of sides (44-47), wherein the sides (44-47) project radially outwards and/or wherein the shape, in particular the height, of some or all sides (44-47) varies along the main axis (A) of the plunger rod (40).

7. Administration device according to any of the preceding claims, **characterized in that** the plunger rod (40) comprises or forms a return stop (44B/45B), preferably wherein the return stop (44B/45B) is formed by a (radial) step/protrusion of the plunger rod (40) and/oder wherein the return stop (44B/45B) is configured to (radially) engage a securing member (90).

8. Administration device according to any of the preceding claims, **characterized in that** the plunger rod (40) comprises or forms actuation intermediate stop (46A/47A) and/or an actuation end stop (44D/45D), preferably wherein the actuation intermediate stop (46A/47A) and/or the actuation end stop (44D/45D) are/is formed by a (radial) step/protrusion and/or an (sharp) edge/enlargement of the plunger rod 40, mostly preferred its cross section, and/or wherein the actuation intermediate stop (46A/47A) and/or the actuation end stop (44D/45D) are/is configured to axially block the plunger rod (40) from being further/completely inserted into the container (10) and/or to axially abut the second actuation part (70) in the administration position and/or in the final position.

9. Administration device according to any of the preceding claims, **characterized in that** the second actuation part (70) comprises an opening (78), wherein the second actuation part (70) is to be actuated to - in particular slidingly - align the opening (78) with the plunger rod (40) such that the plunger rod (40) can move through the opening (78).

10. Administration device according to any of the preceding claims, **characterized in that** the first actuation part (60) can be - in particular manually - actuated in order to move the plunger rod (40) from the initial position to the administration position and subsequently and/or after actuating the second actuation part (70) from the administration position to the final position.

11. Administration device according to any of the preceding claims, **characterized in that** the first actuation part (60) is to be actuated before actuating the second actuation part (70) - in particular for the first time - in order to move the plunger rod (40) from the initial position to the administration position and/or to unblock the second actuation part (70) and/or **in that** the first actuation part (60) is to be actuated again and/or after actuating the second actuation part (70) in order to move the plunger rod (40) from the administration position to the final position.

12. Administration device according to any of the preceding claims, **characterized in that** the second actuation part (70) is blocked in the initial position and/or before reaching the administration position and/or **in that** the second actuation part (70) can be only actuated, in particular for the first time, upon actuation of the first actuation part (60).

13. Administration device according to any of the preceding claims, **characterized in that** the second actuation part (70) is configured to axially block the plunger rod (40) and/or the first actuation part (60) in the administration position of the plunger rod (40), wherein the second actuation part (70) is to be actuated, in particular slided relative to the retainer (20), in order to unblock the plunger rod (40) and/or the first actuation part (60) and/or such that the first actuation part (60) can be actuated, in particular for the second time and/or for administrating of the fluid (11), and/or such that the plunger rod (40) can be moved from the administration position to the final position.

14. Administration device according to any of the preceding claims, **characterized in that** the actuation mechanism comprises a securing member (90) for securing the plunger rod (40) and/or the first actuation part (60), preferably wherein the securing member (90) is coupled loosely and/or with radial play to the plunger rod (40) in the initial position and/oder wherein the securing member (90) is radially pretensioned against and/or tightly coupled to the plunger rod (40) during movement of the plunger rod (40) from the initial position towards the administration position and/or wherein the securing member (90) engages the plunger rod (40) and/or is configured to block a back-movement of the plunger rod (40) and/or the first actuation part (60), at least in the initial position.

15. Administration device according to claim 14, **characterized in that** the plunger rod (40) comprises or forms a ramp (44C) for the securing member (90) and/or **in that** the plunger rod (40), in particular a ramp (44C) thereof, is configured to - in particular further - tension the securing member (90) upon actuating the first actuation part (60) and/or during movement of the plunger rod (40) from the initial position towards the administration position.

16. Administration device according to claim 14 or 15, **characterized in that** the plunger rod (40) comprises an indentation (45C) for the securing member (90) and/or **in that** the plunger rod (40), in particular an indentation (45C) thereof, is configured to at least partially, preferably completely, and/or audibly release the securing member (90) upon actuating the first actuation part (60) and/or in such a way that an audible and/or haptic feedback is provided.
